# EUROPEAN PATENT SPECIFICATION

(11) **EP 3 649 928 B1**
(45) Date of publication and mention of the grant of the patent: **19.03.2025**
(21) Application number: 19208519.9
(22) Date of filing: 12.11.2019
(51) Int. Cl.: A61B 5/021, A61B 5/0295, A61B 5/00, A61B 5/022, A61B 5/026

(54) **BLOOD PRESSURE MEASUREMENT**
MESSUNG DES BLUTDRUCKS
MESURE DE LA PRESSION ARTÉRIELLE

(30) Priority: 12.11.2018 KR 20180138379
(43) Date of publication of application: 13.05.2020
(73) Proprietor: Samsung Electronics Co., Ltd., Gyeonggi-do 16677 (KR)
(72) Inventor: PARK, Sang Yun, Hwaseong-si, Gyeonggi-do (KR); CHOI, Jin Woo, Suwon-si, Gyeonggi-do (KR); KANG, Jae Min, Seoul (KR); KIM, Youn Ho, Hwaseong-si, Gyeonggi-do (KR); CHOI, Chang Mok, Suwon-si, Gyeonggi-do (KR)
(74) Representative: Grünecker Patent- und Rechtsanwälte PartG mbB

(56) References cited:
- EP-A1- 3 342 336
- US-A1- 2001 012 917
- YONGBO LIANG ET AL: "Photoplethysmography and Deep Learning: Enhancing Hypertension Risk Stratification", BIOSENSORS, vol. 8, no. 4, 26 October 2018 (2018-10-26), pages 1 - 13, XP055678541, DOI: 10.3390/bios8040101
- FOROUZANFAR MOHAMAD ET AL: "Oscillometric Blood Pressure Estimation: Past, Present, and Future", IEEE REVIEWS IN BIOMEDICAL ENGINEERING, vol. 8, 15 May 2015 (2015-05-15), pages 44 - 63, XP011666732, ISSN: 1937-3333, [retrieved on 20150817], DOI: 10.1109/RBME.2015.2434215

## Description

### BACKGROUND

### 1. Field

The disclosure relates to technology for measuring blood pressure.

### 2. Description of Related Art

Invasive and non-invasive methods are currently used to measure blood pressure. Invasive methods are generally used for monitoring high-risk patients in an operation room or an intensive care unit. However, the invasive methods involve complicated preparation and procedures, and may cause complications, such as tissue damage, and the like, due to infections and vascular occlusions. Furthermore, the invasive methods are mostly used for critically ill patients, and the use of invasive methods should be managed with careful attention.

Accordingly, non-invasive methods are mainly used for ordinary measurements, but the non-invasive methods have reduced accuracy of measurement as compared to the invasive methods.
The publication of YONGBO LIANG ET AL, titled "Photoplethysmography and Deep Learning: Enhancing Hypertension Risk Stratification", BIOSENSORS, vol. 8, no. 4, dated 26 October 2018, pages 1-13, refers to a blood pressure which is a basic physiological parameter in the cardiovascular circulatory system. Long-term abnormal blood pressure can lead to various cardiovascular diseases, making the early detection and assessment of hypertension profoundly significant for the prevention and treatment of cardiovascular diseases. It has been investigated whether or not deep learning can provide better results for hypertension risk stratification when compared to the classical signal processing and feature extraction methods. A deep learning method for the classification and evaluation of hypertension was tested using photoplethysmography, PPG, signals based on the continuous wavelet transform (using Morse) and pretrained convolutional neural network (using GoogLeNet). The tested deep method achieved higher accuracy for hypertension risk stratification when compared to the classical signal processing and feature extraction method. Additionally, the method achieved comparable results to another approach that requires electrocardiogram and PPG signals.
EP 3 342 336 A1 refers to a touch-type blood pressure measurement apparatus and method. A touch-type blood pressure measurement apparatus is provided. The touch-type blood pressure measurement apparatus includes a touch sensor configured to generate a contact area signal when a finger of a user is in contact with the touch sensor, at least one photoplethysmogram, PPG, sensor configured to generate a PPG signal of the user while the finger is in contact with the touch sensor, a force sensor configured to generate a touch force signal of the finger in contact with the touch sensor, and a controller configured to obtain a blood pressure of the user based on the contact area signal, the PPG signal, and the touch force signal.

### Summary

It is the object of the present invention to provide an improved blood pressure measuring apparatus and improved blood pressure measuring method and related computer-readable medium.

This object is solved by the subject matter of the independent claims.

Preferred embodiments are defined by the dependent claims.

Additional aspects will be set forth in part in the description which follows and, in part, will be apparent from the description, or may be learned by practice of the presented embodiments.

In accordance with an aspect of the disclosure, a blood pressure measuring apparatus includes a pulse wave sensor configured to detect a pulse wave signal of an object, a pressure sensor configured to detect a contact pressure signal corresponding to a contact pressure between the object and the pulse wave sensor, and a processor configured to generate a pulse wave image based on the detected pulse wave signal of the object, generate a contact pressure image based on the detected contact pressure signal corresponding to the contact pressure between the object and the pressure sensor, and estimate a blood pressure of the object based on the generated pulse wave image, the generated contact pressure image, and a blood pressure estimation model.

The pulse wave signal may be a photoplethysmogram signal.

The pulse wave sensor may detect a plurality of pulse wave signals of different wavelengths.

The pressure sensor may detect a contact force signal and a contact area signal corresponding to a contract force and a contract area, respectively, between the object and the pulse wave sensor, and obtain the contact pressure signal based on the contact force signal and the contact area signal.

The processor may generate the pulse wave image by converting the pulse wave signal into an image using at least one of Wavelet Transform, Short Time Fourier transform, and Wigner-Ville distribution.

The processor may generate the contact pressure image by converting the detected contact pressure signal into a gradient image.

The processor may process the pulse wave image and the contact pressure image to have a same time domain scale.

The blood pressure estimation model may be a model based on a convolutional neural network.

The blood pressure estimation model may be generated by machine learning based on training data, and the training data may include pulse wave image data, contact pressure image data, and blood pressure data corresponding thereto (not according to the invention).

The blood pressure measuring apparatus may further include a contact image sensor configured to obtain a plurality of contact images of the object for a time frame.

The processor may convert the obtained plurality of contact images for the time frame into a single contact image which represents a change in a contact image over the time frame, and estimate the blood pressure of the object based on the single contact image.

The processor may perform *n*th-order differentiation on the detected pulse wave signal to obtain an *n*th-order differentiated pulse wave signal, convert the obtained *n*th-order differentiated pulse wave signal into an image to generate an *n*th-order differentiated pulse wave image, and estimate the blood pressure of the object based on the generated *n*th-order differentiated pulse wave image. And *n* may be a natural number.

The processor may generate guide information that instructs a user to increase or decrease the contact pressure between the object and the pressure sensor based on the detected contact pressure signal, and provide, via an output device, the generated guide information to the user.

In accordance with an aspect of the disclosure, a blood pressure measuring method comprises detecting a pulse wave signal of an object, detecting a contact pressure signal corresponding to a contact pressure between the object and a pulse wave sensor, generating a pulse wave image based on the detected pulse wave signal, generating a contact pressure image based on the detected contact pressure signal, and estimating a blood pressure of the object based on the generated pulse wave image, the generated contact pressure image, and a blood pressure estimation model.

The pulse wave signal may be a photoplethysmogram signal.

The detecting of the pulse wave signal may include detecting a plurality of pulse wave signals of different wavelengths.

The detecting of the contact pressure signal may include detecting a contact force signal corresponding to a contact force between the object and the pulse wave sensor, detecting a contact area signal corresponding to a contact area between the object and the pulse wave sensor, and obtaining the contact pressure signal based on the contact force signal and the contact area signal.

The generating of the pulse wave image may include generating the pulse wave image by converting the detected pulse wave signal into an image using at least one of Wavelet Transform, Short Time Fourier transform, and Wigner-Ville distribution.

The generating of the contact pressure image may include generating the contact pressure image by converting the detected contact pressure signal into a gradient image.

The blood pressure measuring method may include generating the pulse wave image and the contact pressure image to have a same time domain scale.

The blood pressure estimation model may be a model based on a convolutional neural network.

The blood pressure estimation model may be generated by machine learning based on training data; and the training data may include pulse wave image data, contact pressure image data, and blood pressure data (not according to the invention).

The blood pressure measuring method may include obtaining a plurality of contact images of the object for a time frame, converting the obtained plurality of contact images for the time frame into a single contact image that represents a change in a contact image over the time frame, wherein the estimating of blood pressure further comprises estimating the blood pressure of the object based on the single contact image.

The blood pressure measuring method may include performing nth-order differentiation on the detected pulse wave signal, to obtain an nth-order differentiated pulse wave signal, and converting the obtained nth-order differentiated pulse wave signal into an image to generate an nth-order differentiated pulse wave image, and the estimating of blood pressure may include estimating the blood pressure of the object based on the generated nth-order differentiated pulse wave image.

The blood pressure measuring method may include generating guide information for instructing a user to increase or decrease the contact pressure between the object and the pulse wave sensor based on the detected contact pressure signal, and providing the generated guide information to the user.

A blood pressure measuring apparatus may include a pulse wave sensor configured to detect a pulse wave signal of an object, a force sensor configured to detect a contact force signal corresponding to a contact force between the object and the pulse wave sensor, an area sensor configured to detect a contact area signal corresponding to a contact area between the object and the pulse wave sensor, and a processor that may generate a pulse wave image based on the detected pulse wave signal of the object, generate a contact force image based on the detected contact force signal corresponding to the contact force between the object and the pulse wave sensor, generate a contact area image based on the detected contact area signal corresponding to the contact area between the object and the pulse wave sensor, and estimate a blood pressure of the object based on the generated pulse wave image, the generated contact force image, the generated contact area image, and a blood pressure estimation model.

The processor may generate the pulse wave image by converting the pulse wave signal into an image using at least one of Wavelet Transform, Short Time Fourier transform, and Wigner-Ville distribution.

The processor may generate the contact force image by converting the detected contact force signal into a gradient image, and generate the contact area image by converting the detected contact area signal into a gradient image.

The blood pressure estimation model may be a model based on a convolutional neural network.

The blood pressure estimation model may be generated by machine learning based on training data; and the training data may include pulse wave image data, contact force image data, contact area image data, and blood pressure data.

In accordance with an aspect of the present invention a blood pressure measuring apparatus includes a cuff which is configured to be worn by an object, a pressure sensor configured to detect a cuff pressure signal corresponding to a pressure between the cuff and the object, and a processor configured to obtain an alternating current (AC) component signal and a direct current (DC) component signal based on the cuff pressure signal, generate an AC component image based on the AC component signal, generate a DC component image based on the DC component signal, and estimate a blood pressure of the object based on the generated AC component image, the generated DC component image, and a blood pressure estimation model.

The processor generates the AC component image by converting the AC component signal into an image using at least one of Wavelet Transform, Short Time Fourier transform, and Wigner-Ville distribution.

The processor generates the DC component image by converting the DC component signal into a gradient image.

The blood pressure estimation model may be a model based on a convolutional neural network.

The blood pressure estimation model is generated by machine learning based on training data; and the training data may include AC component image data, DC component image data, and blood pressure data.

In accordance with an aspect of the present invention, a blood pressure measuring method includes, based on a cuff being placed on an object, detecting a cuff pressure signal corresponding to a pressure between the cuff and the object, extracting an alternating current (AC) component signal and a direct current (DC) component signal based on the cuff pressure signal, generating an AC component image based on the extracted AC component signal, generating a DC component image based on the extracted DC component signal, and estimating a blood pressure of the object based on the generated AC component image, the generated DC component image, and a blood pressure estimation model.

The generating of the AC component image includes generating the AC component image by converting the AC component signal into an image using at least one of Wavelet Transform, Short Time Fourier transform, and Wigner-Ville distribution.

The generating of the DC component image includes generating the DC component image by converting the DC component signal into a gradient image.

The blood pressure estimation model may be a model based on a convolutional neural network.

The blood pressure estimation model is generated by machine learning based on training data; and the training data may include AC component image data, DC component image data, and blood pressure data.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram illustrating an example of a blood pressure measuring apparatus according to an embodiment;
FIG. 2 is a block diagram illustrating an example of a processor according to an embodiment;
FIG. 3 is a diagram illustrating an example of generating a pulse wave image according to an embodiment;
FIG. 4 is a diagram illustrating an example of generating a contact pressure image according to an embodiment;
FIG. 5 is a block diagram illustrating another example of a processor according to an embodiment;
FIG. 6 is a block diagram illustrating an example of a pulse wave sensor according to an embodiment;
FIG. 7 is a block diagram illustrating another example of a pulse wave sensor according to an embodiment;
FIG. 8 is a block diagram illustrating yet another example of a pulse wave sensor according to an embodiment;
FIG. 9 is a block diagram illustrating another example of a blood pressure measuring apparatus according to an embodiment;
FIG. 10 is a block diagram illustrating an example of a processor according to an embodiment;
FIGS. 11 and 12 are diagrams illustrating examples of converting a plurality of contact images for a time frame into a single contact image according to an embodiment;
FIG. 13 is a block diagram illustrating another example of a processor according to an embodiment;
FIG. 14 is a block diagram illustrating yet another example of a blood pressure measuring apparatus according to an embodiment;
FIG. 15 is a flowchart illustrating an example of a blood pressure measuring method according to an embodiment;
FIG. 16 is a flowchart illustrating another example of a blood pressure measuring method according to an embodiment;
FIG. 17 is a flowchart illustrating yet another example of a blood pressure measuring method according to an embodiment;
FIG. 18 is a flowchart illustrating still another example of a blood pressure measuring method according to an embodiment;
FIG. 19 is a flowchart illustrating still another example of a blood pressure measuring method according to an embodiment;
FIG. 20 is a block diagram illustrating still another example of a blood pressure measuring apparatus according to an embodiment;
FIG. 21 is a block diagram illustrating an example of a processor according to an embodiment;
FIG. 22 is a flowchart illustrating still another example of a blood pressure measuring method according to an embodiment;
FIG. 23 is a block diagram illustrating a blood pressure measuring apparatus according to the present invention;
FIG. 24 is a block diagram illustrating an example of a processor according to an embodiment; and
FIG. 25 is a flowchart illustrating a blood pressure measuring method according to the present invention.

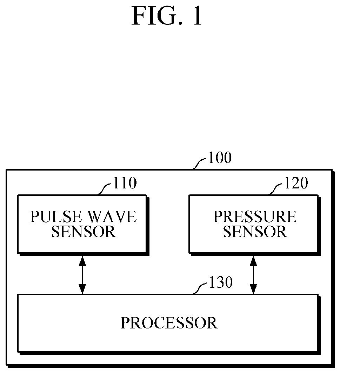

The examples and embodiments according to figures 1 - 22 are not according to the invention.

Throughout the drawings and the detailed description, unless otherwise described, the same drawing reference numerals may refer to the same elements, features, and structures. The relative size and depiction of these elements may be exaggerated for clarity, illustration, and convenience.

### DETAILED DESCRIPTION

Hereinafter, embodiments of the present disclosure will be described in detail with reference to the accompanying drawings. It should be noted that wherever possible, the same reference symbols may refer to the same elements, features, and structures although illustrated in other drawings. In the following description, a detailed description of known functions and configurations incorporated herein may be omitted so as to not obscure the subject matter of the present disclosure.

Process steps described herein may be performed differently from a specified order, unless a specified order is clearly stated in the context of the disclosure. That is, each step may be performed in a specified order, substantially concurrently, in a reverse order, or in a different order.

Further, the terms used throughout this specification are defined in consideration of the functions according to exemplary embodiments, and can be varied according to a purpose of a user or manager, precedent, etc. Therefore, definitions of the terms should be made on the basis of the overall context.

It will be understood that, although the terms "first," "second," etc. may be used herein to describe various elements, these elements might not be limited by these terms. These terms may be used to distinguish one element from another. Any references to the singular forms of terms may include the plural forms of terms unless expressly stated otherwise. In the present disclosure, it should be understood that terms such as "including," "having," etc., may indicate the existence of the features, numbers, steps, actions, components, parts, or combinations thereof disclosed in the specification, and might not preclude the possibility that one or more other features, numbers, steps, actions, components, parts, or combinations thereof may exist or may be added.

Further, components described in the specification may be discriminated according to functions mainly performed by the components. That is, two or more components may be integrated into a single component. Furthermore, a single component may be separated into two or more components. Moreover, each component may additionally perform some or all of a function executed by another component in addition to the main function thereof. Some or all of the main function of each component may be carried out by another component. Each component may be implemented in hardware, software, or a combination of both.

FIG. 1 is a block diagram illustrating an example of a blood pressure measuring apparatus according to an embodiment. The blood pressure measuring apparatus 100 of FIG. 1 is an apparatus for non-invasively measuring blood pressure of an object, and may be embedded in an electronic device or may be enclosed in a housing to be provided as a separate device. Examples of the electronic devices may include a cellular phone, a smartphone, a tablet personal computer (PC), a laptop computer, a personal digital assistant (PDA), a portable multimedia player (PMP), a navigation device, an MP3 player, a digital camera, a wearable device, and the like; and examples of the wearable device may include a wristwatch-type wearable device, a wristband-type wearable device, a ring-type wearable device, a waist belt-type wearable device, a necklace-type wearable device, an ankle band-type wearable device, a thigh band-type wearable device, a forearm band-type wearable device, and the like. However, the electronic device is not limited to the above examples, and the wearable device is neither limited thereto.

Referring to FIG. 1, the blood pressure measuring apparatus 100 includes a pulse wave sensor 110, a pressure sensor 120, and a processor 130.

The pulse wave sensor 110 may detect a pulse wave signal of an object. In an embodiment, the pulse wave sensor 110 may detect at least one pulse wave signal at different wavelengths. That is, the pulse wave sensor 110 may detect a pulse wave signal of a single wavelength, or may detect a plurality of pulse wave signals of different wavelengths. In this case, the pulse wave signal may be a photoplethysmogram (PPG) signal.

For example, based on an object contacting the pulse wave sensor 110, the pulse wave sensor 110 may emit light towards the object, and may detect light reflected by the object to detect at least one pulse wave signal. In this case, light emitted towards the object may be visible light or near infrared light.

The pressure sensor 120 may detect a contact pressure signal corresponding to a contact pressure between the object and the pulse wave sensor 110. In an embodiment, the pressure sensor 120 may detect a contact force signal corresponding to a contact force between the object and the pulse wave sensor 110, and may obtain a contact pressure signal by dividing the contact force signal by a predetermined area. The predetermined area may be pre-stored as a default value in the blood pressure measuring apparatus 100. In another embodiment, the pressure sensor 120 may detect a contact force signal and a contact area signal corresponding to a contact force and a contact area, respectively, between the object and the pulse wave sensor 110, and may obtain a contact pressure signal by dividing the measured contact force signal by the measured contact area signal. To this end, the pressure sensor 120 may include a force sensor, a barometer sensor, an acceleration sensor, a piezoelectric film, a load cell, a radar, a strain gauge, a contact area sensor, and the like.

The processor 130 may control the overall operation of the blood pressure measuring apparatus 100.

Based on the object contacting the pulse wave sensor 110, the processor 130 may control the pulse wave sensor 110 to detect a pulse wave signal of the object, and may control the pressure sensor 120 to detect a contact pressure signal corresponding to a contact pressure between the object and the pulse wave sensor 110.

Further, the processor 130 may estimate blood pressure of the object based on the pulse wave signal and the contact pressure signal. For example, the processor 130 may convert each of the pulse wave signal and the contact pressure signal into an image to generate a pulse wave image and a contact pressure image, and may estimate blood pressure of the object by using the generated pulse wave image and contact pressure image.

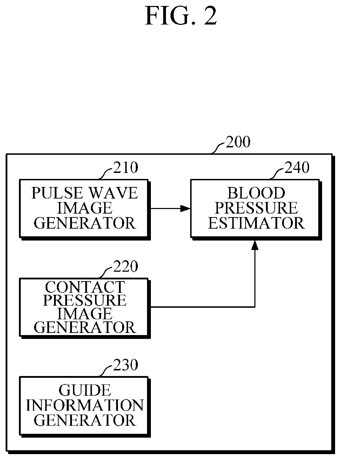

Hereinafter, embodiments of the processor 130 will be described in detail with reference to FIGS. 2 to 5.

FIG. 2 is a block diagram illustrating an example of a processor according to an embodiment. The processor 200 of FIG. 2 may be an example of the processor 130 of FIG. 1.

Referring to FIG. 2, the processor 200 includes a pulse wave image generator 210, a contact pressure image generator 220, a guide information generator 230, and a blood pressure estimator 240.

The pulse wave image generator 210 may convert a pulse wave signal into an image by performing frequency domain conversion to generate a pulse wave image. The pulse wave signal may be a periodic signal having a shape which contains significant information. In an embodiment, the pulse wave image generator 210 may generate the pulse wave image by converting a pulse wave signal into an image using one of Wavelet Transform, Short Time Fourier transform, and Wigner-Ville distribution. In this case, the generated pulse wave image may be represented in a time domain and a frequency domain. That is, a horizontal axis of the pulse wave image represents a time domain which reflects physical properties with respect to time; and a vertical axis of the pulse wave image represents a frequency domain which reflects physical properties with respect to frequency. However, the pulse wave image is not limited thereto, and a horizontal axis may represent a frequency domain reflecting physical properties with respect to frequency, and a vertical axis may represent a time domain reflecting physical properties with respect to time.

The contact pressure image generator 220 may generate a contact pressure image by converting a contact pressure signal into an image. The contact pressure signal may be a non-periodic signal having an intensity which contains significant information. In an embodiment, the contact pressure image generator 220 may generate the contact pressure image by converting a contact pressure signal into a gradient image. In this case, the generated contact pressure image may be represented in a time domain. That is, one axis (e.g., either a horizontal axis or a vertical axis) of the contact pressure image may represent a time domain reflecting physical properties with respect to time.

Based on the measured contact pressure signal, the guide information generator 230 may generate guide information for instructing a user to increase or decrease a contact pressure between an object and the pulse wave sensor for measuring blood pressure. Then, the guide information generator 230 may provide the generated guide information to a user via an output device. For example, the guide information generator 230 may compare the measured contact pressure signal with a target pressure signal which linearly increases or decreases; and in response to the measured contact pressure signal being greater than the target pressure signal, the guide information generator 230 may generate guide information for instructing a user to decrease the contact pressure, and in response to the measured contact pressure signal being less than the target pressure signal, the guide information generator 230 may generate guide information for instructing the user to increase the contact pressure. Then, the guide information generator 230 may provide the generated guide information to the user via an output device. In this case, examples of the output device may include various output devices such as a visual output device, an audio output device, a haptic output device, and the like.

The blood pressure estimator 240 may estimate blood pressure of the object based on the pulse wave image and the contact pressure image. In this case, the blood pressure estimator 240 may use a pre-generated blood pressure estimation model. The blood pressure estimation model may be a model which is based on a convolutional neural network and is pre-generated by machine learning using pulse wave image data, contact pressure image data, and blood pressure data corresponding thereto as training data, and may be stored in an internal or external memory of the processor 200.

In an embodiment, before estimating blood pressure, the blood pressure estimator 240 may process the pulse wave image and the contact pressure image so that the pulse wave image and the contact pressure image may have the same size. For example, the blood pressure estimator 240 may process the pulse wave image and the contact pressure image so that the pulse wave image and the contact pressure image may have the same time domain scale.

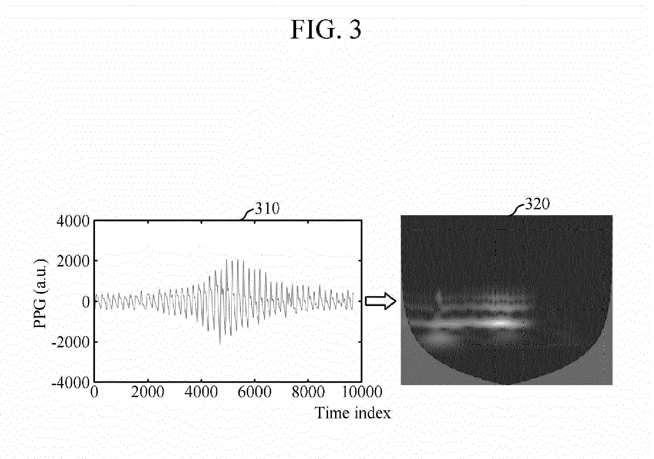

FIG. 3 is a diagram illustrating an example of generating a pulse wave image according to an embodiment. FIG. 3 is an example of generating a pulse wave image by converting a PPG signal using Wavelet Transform.

Referring to FIGS. 2 and 3, the pulse wave image generator 210 may convert a pulse wave signal 310 using Wavelet Transform to generate a pulse wave image 320. The pulse wave signal 310 is a periodic signal having a shape which contains significant information. In the pulse wave signal 310, physical properties with respect to time and physical properties with respect to frequency may be important factors in estimating blood pressure. Accordingly, the pulse wave image generator 210 may generate the pulse wave image 320 by performing frequency domain conversion such as Wavelet Transform, so that physical properties with respect to time and physical properties with respect to frequency of the pulse wave signal 310 may be represented. In this case, a shape of an envelope or a combination of high-frequency components, which form the shape of the pulse wave signal 310, may be reflected in the generated pulse wave image 320.

As illustrated in FIG. 3, a horizontal axis of the pulse wave image 320 represents a time domain reflecting physical properties with respect to time, and a vertical axis of the pulse wave image 320 represents a frequency domain reflecting physical properties with respect to frequency.

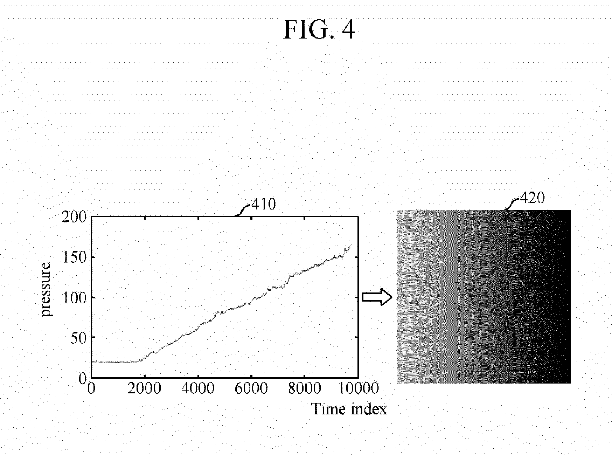

FIG. 4 is a diagram illustrating an example of generating a contact pressure image according to an embodiment. FIG. 4 illustrates an example of generating a contact pressure image by converting a contact pressure signal into a gradient image.

Referring to FIGS. 2 and 4, the contact pressure image generator 220 may convert a contact pressure signal 410 into a gradient image to generate a contact pressure image 420. The contact pressure signal 410 may be a non-periodic signal having an intensity which contains significant information, and physical properties with respect to time of the contact pressure signal 410 may be a relatively important factor in estimating blood pressure. Accordingly, the contact pressure image generator 220 may generate the contact pressure image 420 by converting the contact pressure signal 410 into a gradient image, so that physical properties with respect to time (e.g., signal intensity over time) of the contact pressure signal 410 may be well represented.

As illustrated in FIG. 4, a horizontal axis of the contact pressure image 420 represents a time domain reflecting physical properties with respect to time.

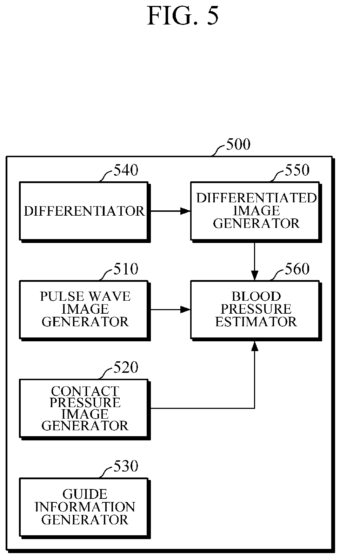

FIG. 5 is a block diagram illustrating another example of a processor according to an embodiment. The processor 500 of FIG. 5 may be another example of the processor 130 of FIG. 1.

Referring to FIG. 5, the processor 500 includes a pulse wave image generator 510, a contact pressure image generator 520, a guide information generator 530, a differentiator 540, a differentiated image generator 550, and a blood pressure estimator 560. Here, the pulse wave image generator 510, the contact pressure image generator 520, and the guide information generator 530 may be substantially similar as the pulse wave image generator 210, the contact pressure image generator 220, and the guide information generator 230 of FIG. 2, such that detailed description thereof may be omitted.

The differentiator 540 may perform nth-order differentiation on a pulse wave signal to obtain an *n*th-order differentiated pulse wave signal, in which *n* may be a natural number.

The differentiated image generator 550 may convert the obtained *n*th-order differentiated pulse wave signal into an image to generate an nth-order differentiated pulse wave image. The pulse wave signal may be a periodic signal having a shape which contains significant information, such that the nth-order differentiated pulse wave signal may also be a periodic signal having a shape which contains significant information. The differentiated image generator 550 may convert the nth-order differentiated pulse wave signal into an image by performing frequency domain conversion on the nth-order differentiated pulse wave signal, to generate an nth-order differentiated pulse wave image. In an embodiment, the differentiated image generator 550 may convert the nth-order differentiated pulse wave signal into an image using at least one of Wavelet Transform, Short Time Fourier transform, and Wigner-Ville distribution, to generate the nth-order differentiated pulse wave image. The generated nth-order differentiated pulse wave image may be represented in a time domain and a frequency domain. That is, a horizontal axis of the nth-order differentiated pulse wave image represents a time domain which reflects physical properties with respect to time; and a vertical axis of the pulse wave image represents a frequency domain which reflects physical properties with respect to frequency. However, the nth-order differentiated pulse wave image is not limited thereto, and a horizontal axis of the nth-order differentiated pulse wave image may be a frequency domain reflecting physical properties with respect to frequency, and a vertical axis thereof may be a time domain reflecting physical properties with respect to time.

The blood pressure estimator 560 may estimate blood pressure of an object based on the pulse wave image, the contact pressure image, and the nth-order differentiated pulse wave image. In this case, the blood pressure estimator 560 may use a pre-generated blood pressure estimation model. The blood pressure estimation model may be a model which is based on a convolutional neural network and is pre-generated by machine learning using pulse wave image data, contact pressure image data, nth-order differentiated pulse wave image data, and blood pressure data corresponding thereto as training data, and may be stored in an internal or external memory of the processor 500.

In an embodiment, before estimating blood pressure, the blood pressure estimator 560 may process the pulse wave image, the contact pressure image, and the nth-order differentiated pulse wave image so that the pulse wave image, the contact pressure image, and the nth-order differentiated pulse wave image may have the same size. For example, the blood pressure estimator 560 may process the pulse wave image, the contact pressure image, and the nth-order differentiated pulse wave image so that the pulse wave image, the contact pressure image, and the nth-order differentiated pulse wave image may have the same time domain scale.

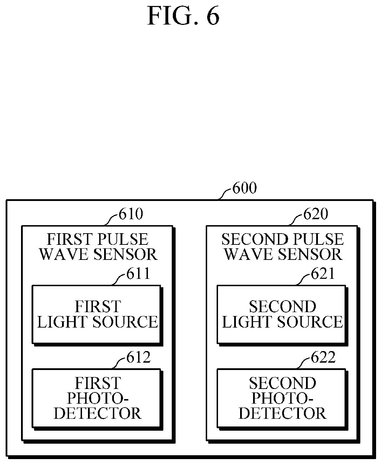

FIG. 6 is a block diagram illustrating an example of a pulse wave sensor. FIG. 6 illustrates an example of measuring two or more pulse wave signals of different wavelengths; and the pulse wave sensor 600 of FIG. 6 may be an example of the pulse wave sensor 110 of FIG. 1.

Referring to FIG. 6, the pulse wave sensor 600 may be formed as an array of pulse wave sensors for measuring a plurality of pulse wave signals of different wavelengths. As illustrated in FIG. 6, the pulse wave sensor 600 includes a first pulse wave sensor 610 and a second pulse wave sensor 620. However, this is merely an example for convenience of explanation, and the number of pulse wave sensors included in the array is not specifically limited.

The first pulse wave sensor 610 includes a first light source 611 which emits light of a first wavelength towards an object; and a first photodetector 612 which detects a first pulse wave signal by detecting light of the first wavelength emitted by the first light source 611 and reflected by the object.

The second pulse wave sensor 620 includes a second light source 621 which emits light of a second wavelength towards an object; and a second photodetector 622 which detects a second pulse wave signal by detecting light of the second wavelength emitted by the second light source 621 and reflected by the object.

In an embodiment, the first light source 611 and the second light source 621 may include a light-emitting diode (LED), an organic light emitting diode (OLED), a Quantum dot light-emitting diode (QLED), a laser diode, a fluorescent body, and the like, but are not limited thereto. Further, the first photodetector 612 and the second photodetector 622 may include a photo diode, a photo transistor (PTr), a charge-coupled device (CCD), a complementary metal-oxide semiconductor (COMS), and the like, but are not limited thereto.

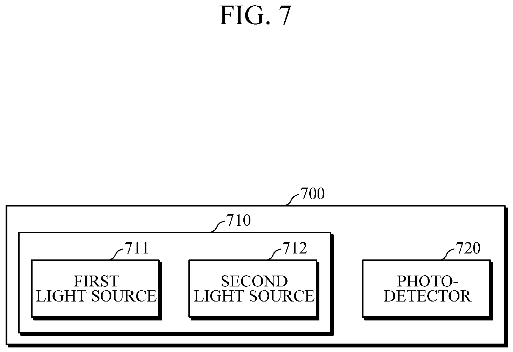

FIG. 7 is a block diagram illustrating another example of a pulse wave sensor. FIG. 7 illustrates an example of detecting two or more pulse wave signals of different wavelengths; and the pulse wave sensor 700 of FIG. 7 may be an example of the pulse wave sensor 110 of FIG. 1.

Referring to FIG. 7, the pulse wave sensor 700 includes a light source part 710 including a first light source 711 and a second light source 712, and a photodetector 720. While FIG. 7 illustrates an example where the light source part 710 includes two light sources 711 and 712, this is merely an example for convenience of explanation, and the number of light sources is not specifically limited.

The first light source 711 may emit light of a first wavelength towards an object, and the second light source 712 may emit light of a second wavelength towards an object. In this case, the first wavelength and the second wavelength may be different from each other.

The photodetector 720 may detect a first pulse wave signal by detecting light of the first wavelength emitted by the first light source 711 and reflected by the object, and may detect a second pulse wave signal by detecting light of the second wavelength emitted by the second light source 712 and reflected by the object.

For example, the first light source 711 and the second light source 712 may be driven in a time-division manner under the control of the processor to sequentially emit light towards the object. In this case, light source driving conditions, such as an emission time, a driving sequence, a current intensity, a pulse duration, and the like, of the first light source 711 and the second light source 712 may be preset. The processor may control driving of each of the light sources 711 and 712 by referring to the preset light source driving conditions.

The photodetector 720 may detect the first pulse wave signal and the second pulse wave signal by sequentially detecting light of the first wavelength and light of the second wavelength, which are sequentially emitted by the first light source 711 and the second light source 712 and reflected by the object.

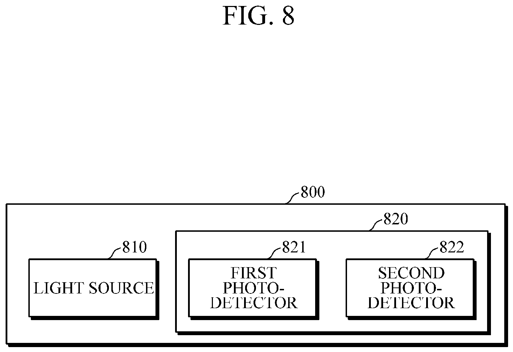

FIG. 8 is a block diagram illustrating yet another example of a pulse wave sensor. FIG. 8 illustrates an example of detecting two or more pulse wave signals of different wavelengths; and the pulse wave sensor 800 of FIG. 8 may be an example of the pulse wave sensor 110 of FIG. 1.

Referring to FIG. 8, the pulse wave sensor 800 includes a light source 810 and a photodetector part 820. The photodetector part 820 includes a first photodetector 821 and a second photodetector 822. While FIG. 8 illustrates an example where the photodetector part 820 includes two photodetectors 821 and 822, this is merely an example for convenience of explanation, and the number of photodetectors is not specifically limited.

The light source 810 may emit light of a predetermined wavelength range towards an object. In this case, the light source 810, which is a single light source, may emit light in a broad wavelength range including a visible light range.

The photodetector part 820 may detect a plurality of pulse wave signals by detecting light in a predetermined wavelength range which is reflected by an object. To this end, the first photodetector 821 and the second photodetector 822, which are included in the photodetector part 820, may have different response characteristics.

For example, the first photodetector 821 and the second photodetector 822 may have different measurement ranges to respond to light of different wavelengths among light beams reflected by an object. Alternatively, a filter may be mounted on a front surface of any one of the first photodetector 821 and the second photodetector 822, or different filters may be mounted on front surfaces of the two photodetectors 821 and 822, so as to respond to light of different wavelengths. In addition, the first photodetector 821 and the second photodetector 822 may be positioned at different distances from the light source 810. In this case, any one of the photodetectors 821 and 822, which is positioned at a relatively short distance from the light source 810, may detect light in a short wavelength range; and the other one, which is positioned at a relatively long distance from the light source 810, may detect light in a long wavelength range.

Embodiments of pulse wave sensors for detecting a plurality of pulse wave signals of different wavelengths are described above in more detail with reference to FIGS. 6 to 8, which are merely examples and the pulse wave sensor is not limited thereto. That is, there may be various numbers and arrangements of the light sources and the photodetectors, and the number and arrangement thereof may vary according to a purpose of use of the pulse wave sensor, the size and shape of an electronic device in which the pulse wave sensor is mounted, and the like.

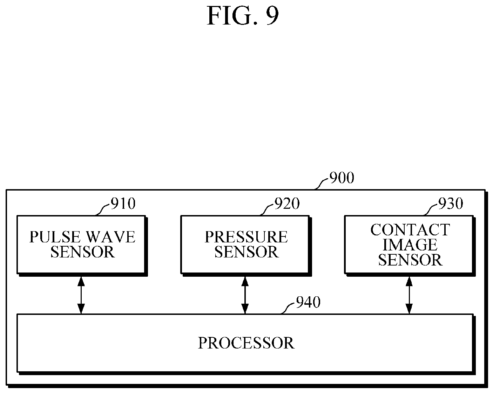

FIG. 9 is a block diagram illustrating another example of a blood pressure measuring apparatus according to an embodiment. The blood pressure measuring apparatus 900 of FIG. 9 is an apparatus for non-invasively measuring blood pressure of an object, and may be embedded in an electronic device or may be enclosed in a housing to be provided as a separate device.

Referring to FIG. 9, the blood pressure measuring apparatus 900 includes a pulse wave sensor 910, a pressure sensor 920, a contact image sensor 930, and a processor 940. Here, the pulse wave sensor 910 and the pressure sensor 920 may be substantially similar as the pulse wave sensor 110 and the pressure sensor 120 of FIG. 1, such that detailed description thereof may be omitted.

The contact image sensor 930 may obtain a plurality of contact images of an object for a time frame. To this end, the contact image sensor 930 may include a touch sensor , a fingerprint sensor, or the like. For example, the contact image sensor 930 may obtain the contact images for a time frame by contouring based on sensor values of a touch sensor or a fingerprint sensor for the time frame.

The processor 940 may control the overall operation of the blood pressure measuring apparatus 900.

Once the object comes into contact with the pulse wave sensor 910, the processor 940 may control the pulse wave sensor 910 to detect a pulse wave signal of the object; may control the pressure sensor 920 to detect a contact pressure signal corresponding to a contact pressure between the object and the pulse wave sensor 910; and may control the contact image sensor 930 to obtain a plurality of contact images for a time frame.

Further, the processor 940 may estimate blood pressure of the object based on the detected pulse wave signal, the detected contact pressure signal, and the obtained plurality of contact images for the time frame. For example, the processor 940 may convert each of the pulse wave signal and the contact pressure signal into an image to generate a pulse wave image and a contact pressure image, may convert the plurality of contact images for the time frame into a single contact image which represents a change in a contact image over the time frame, and may estimate blood pressure of the object by using the pulse wave image, the contact pressure image, and the single contact image.

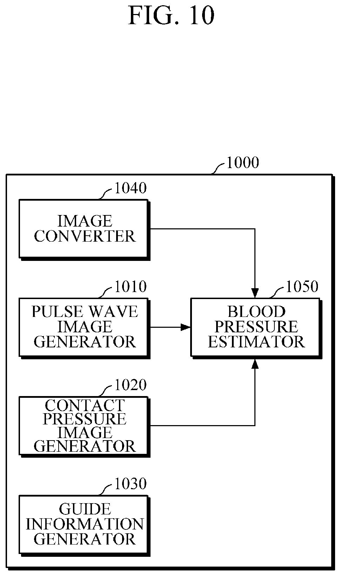

Hereinafter, embodiments of the processor 940 will be described in detail with reference to FIGS. 10 to 13.

FIG. 10 is a block diagram illustrating an example of a processor according to an embodiment. The processor 1000 of FIG. 10 may be an example of the processor 940 of FIG. 9.

Referring to FIG. 10, the processor 1000 includes a pulse wave image generator 1010, a contact pressure image generator 1020, a guide information generator 1030, an image converter 1040, and a blood pressure estimator 1050. Here, the pulse wave image generator 1010, the contact pressure image generator 1020, and the guide information generator 1030 may be substantially similar as the pulse wave image generator 210, the contact pressure image generator 220, and the guide information generator 230 of FIG. 2, such that detailed description thereof may be omitted.

The image converter 1040 may convert a plurality of contact images for a time frame into a single contact image which represents a change in a contact image over the time frame. For example, the image converter 1040 may convert a plurality of contact images for a time frame into a single contact image by: dividing a contact image for a time frame into a plurality of portions, arranging the portions in a predetermined arrangement order to generate arranged images of the contact image for the time frame, and arranging the generated arranged images in a time-sequential order.

The blood pressure estimator 1050 may estimate blood pressure of an object based on the pulse wave image, the contact pressure image, and the single contact image. In this case, the blood pressure estimator 1050 may use a pre-generated blood pressure estimation model. The blood pressure estimation model may be a model which is based on a convolutional neural network and is pre-generated by machine learning using pulse wave image data, contact pressure image data, single contact image data, and blood pressure data corresponding thereto as training data, and may be stored in an internal or external memory of the processor 1000.

In an embodiment, before estimating blood pressure, the blood pressure estimator 1050 may process the pulse wave image, the contact pressure image, and the single contact image so that the pulse wave image, the contact pressure image, and the single contact image may have the same size. For example, the blood pressure estimator 1050 may process the pulse wave image, the contact pressure image, and the single contact image so that the pulse wave image, the contact pressure image, the single contact image may have the same time domain scale.

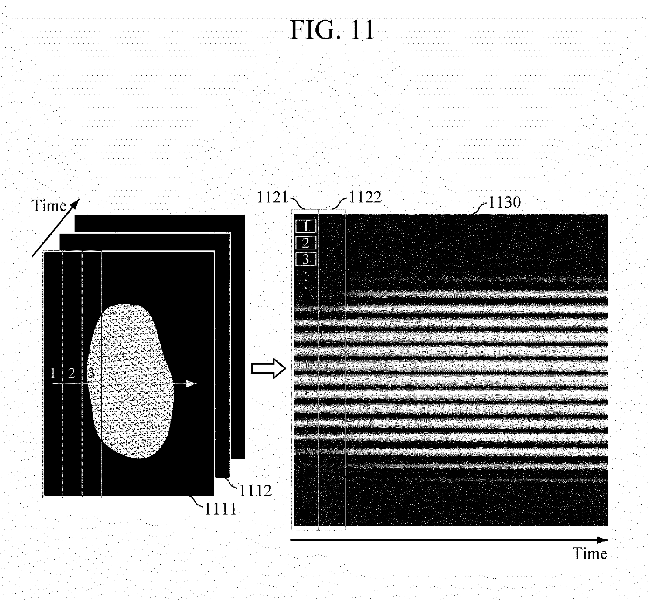

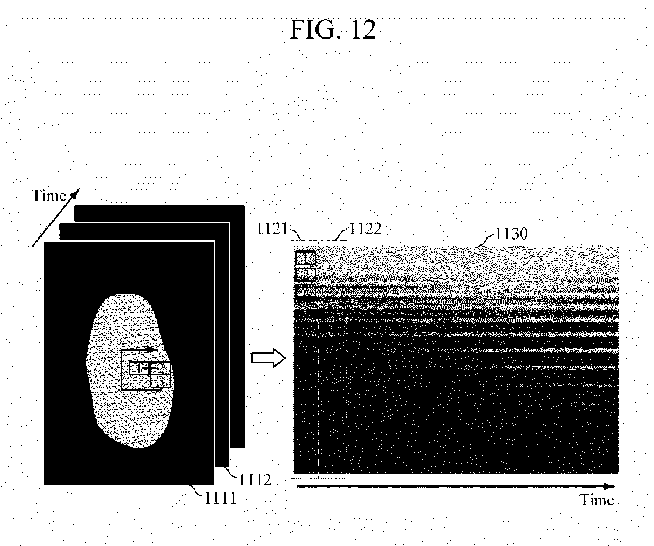

FIGS. 11 and 12 are diagrams illustrating examples of converting a plurality of contact images for a time frame into a single contact image according to an embodiment.

Referring to FIGS. 10 to 12, the image converter 1040 may convert a plurality of contact images 1111 and 1112 for a time frame into a single contact image 1130 which represents a change in a contact image over the time frame. In the illustrated example, the image converter 1040 may divide a first contact image 1111 of the time frame into a plurality of portions 1, 2, and 3, and may sequentially arrange the portions in a predetermined arrangement order from top to bottom, to generate an arranged image 1121 of the first contact image 1111 for the time frame. Further, the image converter 1040 may divide the second contact image 1112 of the time frame into a plurality of portions, and may sequentially arrange the portions in a predetermined arrangement order from top to bottom, to generate an arranged image 1122 of the second contact image 1112 for the time frame. In this manner, the image converter 1040 may generate the arranged images of all the contact images for the time frame, and may arrange the generated arranged images 1121 and 1122 in a time-sequential order, to generate the single contact image 1130.

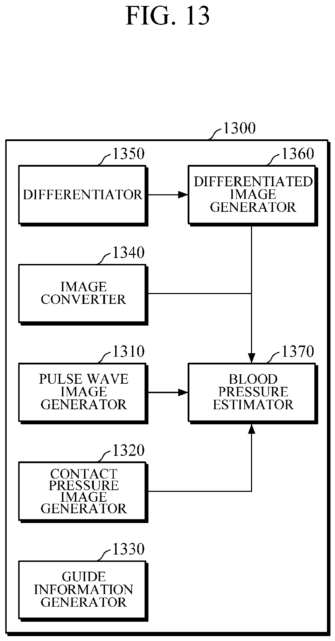

FIG. 13 is a block diagram illustrating another example of a processor according to an embodiment. The processor 1300 of FIG. 13 may be an example of the processor 940 of FIG. 9.

Referring to FIG. 13, the processor 1300 includes a pulse wave image generator 1310, a contact pressure image generator 1320, a guide information generator 1330, an image converter 1340, a differentiator 1350, a differentiated image generator 1360, and a blood pressure estimator 1370. Here, the pulse wave image generator 1310, the contact pressure image generator 1320, the guide information generator 1330, and the image converter 1340 may be substantially similar as the pulse wave image generator 1010, the contact pressure image generator 1020, the guide information generator 1030, and the image converter 1040 respectively of FIG. 10, such that detailed description thereof may be omitted. Further, the differentiator 1350 and the differentiated image generator 1360 may be substantially similar as the differentiator 540 and the differentiated image generator 550 of FIG. 5, such that detailed description thereof may be omitted.

The blood pressure estimator 1370 may estimate blood pressure of an object based on the pulse wave image, the contact pressure image, the single contact image, and the nth-order differentiated pulse wave image. In this case, the blood pressure estimator 1370 may use a pre-generated blood pressure estimation model. The blood pressure estimation model may be a model which is based on a convolutional neural network and is pre-generated by machine learning using pulse wave image data, contact pressure image data, single contact image data, nth-order differentiated pulse wave image data, and blood pressure data corresponding thereto as training data, and may be stored in an internal or external memory of the processor 1300.

In an embodiment, before estimating blood pressure, the blood pressure estimator 1370 may process the pulse wave image, the contact pressure image, the single contact image, and the nth-order differentiated pulse wave image so that the pulse wave image, the contact pressure image, the single contact image, and the nth-order differentiated pulse wave image may have the same size. For example, the blood pressure estimator 1370 may process the pulse wave image, the contact pressure image, the single contact image, and the nth-order differentiated pulse wave image so that the pulse wave image, the contact pressure image, the single contact image, and the nth-order differentiated pulse wave image may have the same time domain scale.

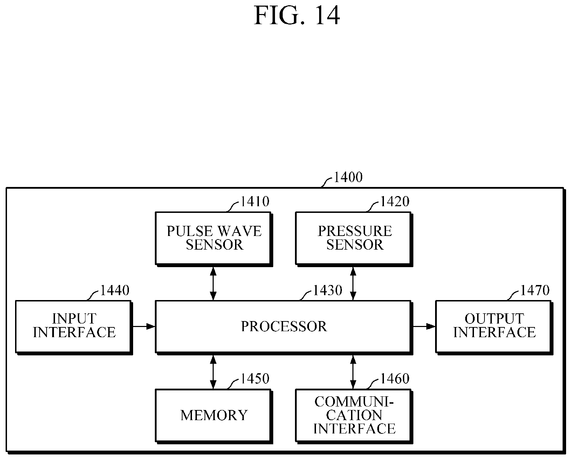

FIG. 14 is a block diagram illustrating yet another example of a blood pressure measuring apparatus according to an embodiment. The blood pressure measuring apparatus 1400 of FIG. 14 is an apparatus for non-invasively measuring blood pressure of an object, and may be embedded in an electronic device or may be enclosed in a housing to be provided as a separate device.

Referring to FIG. 14, the blood pressure measuring apparatus 1400 includes a pulse wave sensor 1410, a pressure sensor 1420, a processor 1430, an input interface 1440, a memory 1450, a communication interface 1460, and an output interface 1470. Here, the pulse wave sensor 1410, the pressure sensor 1420, and the processor 1430 may be substantially similar as the pulse wave sensor 110, the pressure sensor 120, and the processor 130 respectively of FIG. 1, such that detailed description thereof may be omitted.

The input interface 1440 may receive input of various operation signals based a user input. In an embodiment, the input interface 1440 may include a keypad, a dome switch, a touch pad (e.g., a static pressure touch pad, a capacitive touch pad, etc.), a jog wheel, a jog switch, a hardware (H/W) button, and the like. Particularly, the touch pad, which forms a layered structure with a display, may be referred to as a touch screen.

The memory 1450 may store programs or commands for operation of the blood pressure measuring apparatus 1400, and may store data input to and output from the blood pressure measuring apparatus 1400. Further, the memory 1450 may store the pulse wave signal, the contact pressure signal, the pulse wave image, the contact pressure image, the blood pressure estimation model, and the like.

The memory 1450 may include at least one storage medium of a flash memory type memory, a hard disk type memory, a multimedia card micro type memory, a card type memory (e.g., a secure digital (SD) memory, an extreme digital (XD) memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like. Further, the blood pressure measuring apparatus 1400 may communicate with an external storage medium, such as web storage and the like, which performs a storage function of the memory 1450 on the Internet.

The communication interface 1460 may perform communication with an external device. For example, the communication interface 1460 may transmit, to the external device, data input to the blood pressure measuring apparatus 1400, data stored in or processed by the blood pressure measuring apparatus 1400, and the like; or may receive, from the external device, various data for estimating blood pressure of an object.

In this case, the external device may be medical equipment using the data input to the blood pressure measuring apparatus 1400, the data stored in or processed by the blood pressure measuring apparatus 1400, and the like, a printer to print out results, or a display to display the results. In addition, the external device may be a digital television (TV), a desktop computer, a cellular phone, a smartphone, a tablet PC, a laptop computer, a PDA, a PMP, a navigation device, an MP3 player, a digital camera, a wearable device, and the like, but is not limited thereto.

The communication interface 1460 may communicate with an external device by using Bluetooth communication, Bluetooth Low Energy (BLE) communication, Near Field Communication (NFC), wireless local area network (WLAN) communication, Zigbee communication, Infrared Data Association (IrDA) communication, wireless fidelity (Wi-Fi) communication, Ultra-Wideband (UWB) communication, Ant+ communication, Wi-Fi Direct (WFD) communication, Radio Frequency Identification (RFID) communication, third generation (3G) communication, fourth generation (4G) communication, fifth generation (5G) communication, and the like. However, this is merely exemplary and is not intended to be limiting.

The output interface 1470 may output the data input to the blood pressure measuring apparatus 1400, the data stored in or processed by the blood pressure measuring apparatus 1400, and the like. In an embodiment, the output interface 1470 may output the data input to the blood pressure measuring apparatus 1400, the data stored in or processed by the blood pressure measuring apparatus 1400, and the like by using at least one of an acoustic method, a visual method, and a tactile method. To this end, the output interface 1470 may include a speaker, a display, a vibrator, and the like.

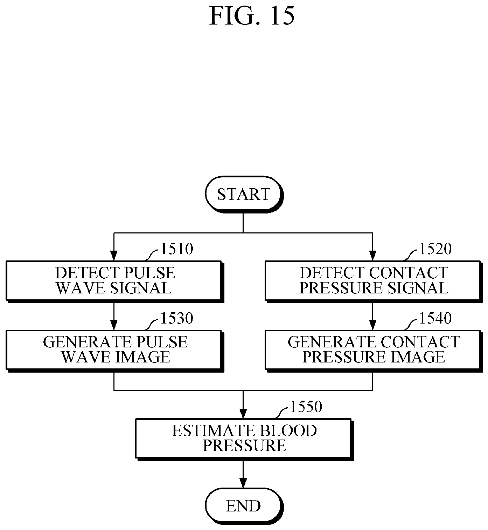

FIG. 15 is a flowchart illustrating an example of a blood pressure measuring method according to an embodiment. The blood pressure measuring method of FIG. 15 may be performed by the blood pressure measuring apparatus 100 of FIG. 1.

Referring to FIG. 15, the blood pressure measuring apparatus may detect a pulse wave signal of an object in operation 1510. In an embodiment, the blood pressure measuring apparatus may detect at least one pulse wave signal of different wavelengths. For example, based on the object contacting a pulse wave sensor, the blood pressure measuring apparatus may detect at least one pulse wave signal of different wavelengths by detecting light reflected by the object.

The blood pressure measuring apparatus may detect a contact pressure signal corresponding to a contact pressure between the object and the pulse wave sensor in operation 1520. In an embodiment, the blood pressure measuring apparatus may detect a contact force signal corresponding to a contact force between the object and the pulse wave sensor, and may obtain a contact pressure signal by dividing the detected contact force signal by a predetermined area. The predetermined area may be pre-stored as a default value in the blood pressure measuring apparatus. In an embodiment, the blood pressure measuring apparatus may detect a contact force signal and a contact area signal corresponding to a contact force and a contact area, respectively, between the object and the pulse wave sensor, and may obtain a contact pressure signal by dividing the measured contact force signal by the measured contact area signal.

The blood pressure measuring apparatus may convert the pulse wave signal into an image by performing frequency domain conversion, to generate a pulse wave image in operation 1530. In an embodiment, the blood pressure apparatus may generate a pulse wave image by converting a pulse wave signal into an image using at least one of Wavelet Transform, Short Time Fourier transform, and Wigner-Ville distribution.

The blood pressure measuring apparatus may convert a contact pressure signal into an image to generate a contact pressure image in operation 1540. In an embodiment, the blood pressure measuring apparatus may generate the contact pressure image by converting the contact pressure signal into a gradient image.

The blood pressure measuring apparatus may estimate blood pressure of an object by using the pulse wave image, the contact pressure image, and a blood pressure estimation model in operation 1550. The blood pressure estimation model may be a model which is based on a convolutional neural network and is pre-generated by machine learning using pulse wave image data, contact pressure image data, and blood pressure data corresponding thereto as training data.

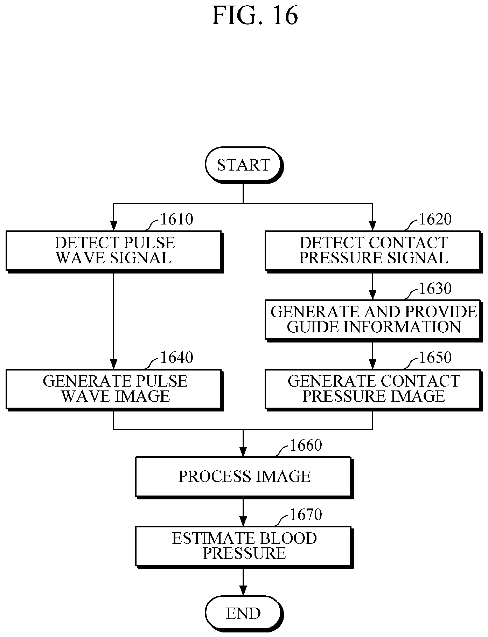

FIG. 16 is a flowchart illustrating another example of a blood pressure measuring method according to an embodiment. The blood pressure measuring method of FIG. 16 may be performed by the blood pressure measuring apparatus 100 of FIG. 1. Operations 1610, 1620, 1640, 1650, and 1670 of FIG. 16 may be substantially similar as the operations 1510, 1520, 1530, 1540, and 1550, respectively of FIG. 15, such that description thereof may be briefly made below.

Referring to FIG. 16, the blood pressure measuring apparatus may detect a pulse wave signal of an object in operation 1610, and may detect a contact pressure signal corresponding to a contact pressure between the object and the pulse wave sensor in operation 1620.

Based on the detected contact pressure signal, the blood pressure measuring apparatus may generate guide information that instructs a user to increase or a decrease a contact pressure between an object and the pulse wave sensor for measuring blood pressure, and may provide the generated guide information to a user via an output device in operation 1630. For example, the blood pressure measuring apparatus may compare the detected contact pressure signal with a target pressure signal which linearly increases or decreases; and in response to the measured contact pressure signal being greater than the target pressure signal, the blood pressure measuring apparatus may generate guide information that instructs the user to decrease the contact pressure, and in response to the measured contact pressure signal being less than the target pressure signal, the blood pressure measuring apparatus may generate guide information that instructs the user to increase the contact pressure. Then, the blood pressure measuring apparatus may provide the generated guide information to a user via an output device.

The blood pressure measuring apparatus may convert the pulse wave signal into an image by performing frequency domain conversion to generate a pulse wave image in operation 1640, and may convert the contact pressure signal into an image to generate a contact pressure image in operation 1650.

The blood pressure measuring apparatus may process the pulse wave image and the contact pressure image in operation 1660, so that the pulse wave image and the contact pressure image may have the same size. For example, the blood pressure measuring apparatus may process the pulse wave image and the contact pressure image so that pulse wave image and the contact pressure image may have the same time domain scale.

The blood pressure measuring apparatus may estimate blood pressure of an object by using the pulse wave image, the contact pressure image, and the blood pressure estimation model in 1670.

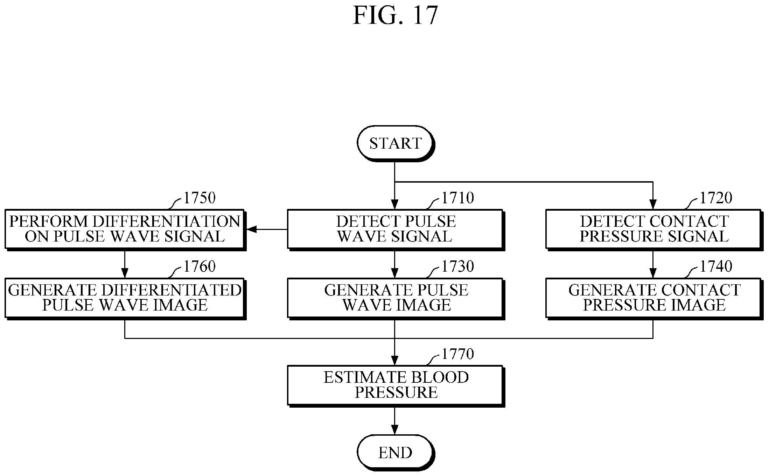

FIG. 17 is a flowchart illustrating yet another example of a blood pressure measuring method according to an embodiment. The blood pressure measuring method of FIG. 17 may be performed by the blood pressure measuring apparatus 100 of FIG. 1. Operations 1710, 1720, 1730, and 1740 of FIG. 17 may be substantially similar as the operations 1510, 1520, 1530, and 1540, respectively, of FIG. 15, such that description thereof may be briefly made below.

Referring to FIG. 17, the blood pressure measuring apparatus may detect a pulse wave signal of an object in operation 1710, and may detect a contact pressure signal corresponding to a contact pressure between the object and the pulse wave sensor in operation 1720.

The blood pressure measuring apparatus may convert the pulse wave signal into an image by performing frequency domain conversion to generate a pulse wave image in operation 1730, and may convert the contact pressure signal into an image to generate a contact pressure image in operation 1740.

The blood pressure measuring apparatus may perform *n*th-order differentiation on the pulse wave signal to obtain an *n*th-order differentiated pulse wave signal in operation 1750, in which *n* may be a natural number.

The blood pressure measuring apparatus may convert the obtained *n*th-order differentiated pulse wave signal into an image by performing frequency domain conversion, to generate an *n*th-order differentiated pulse wave image in operation 1760. In an embodiment, the blood pressure measuring apparatus may generate the *n*th-order differentiated pulse wave image by converting the *n*th-order differentiated pulse wave signal into an image using at least one of Wavelet Transform, Short Time Fourier transform, and Wigner-Ville distribution.

The blood pressure measuring apparatus may estimate blood pressure of an object by using the pulse wave image, the contact pressure image, and the *n*th-order differentiated pulse wave image, and a blood pressure estimation model in operation 1770. The blood pressure estimation model may be a model which is based on a convolutional neural network and is pre-generated by machine learning using pulse wave image data, contact pressure image data, *n*th-order differentiated pulse wave image data, and blood pressure data corresponding thereto as training data.

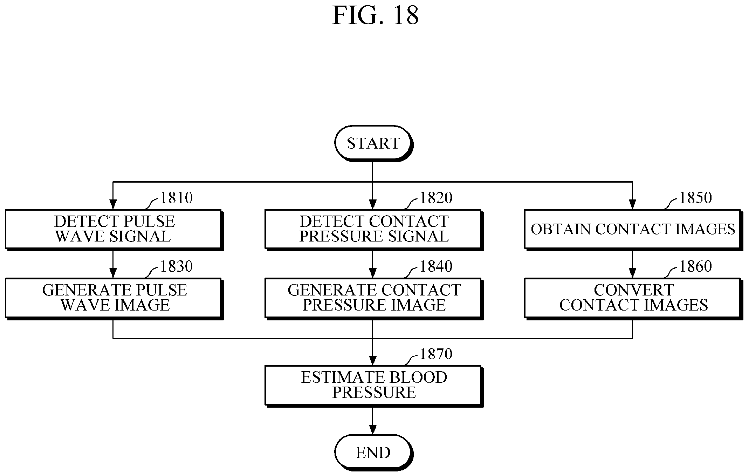

FIG. 18 is a flowchart illustrating still another example of a blood pressure measuring method according to an embodiment. The blood pressure measuring method of FIG. 18 may be performed by the blood pressure measuring apparatus 900 of FIG. 9. Operations 1810, 1820, 1830, and 1840 of FIG. 18 may be substantially similar as the operations 1510, 1520, 1530, and 1540, respectively of FIG. 15, such that description thereof may be briefly made below.

Referring to FIG. 18, the blood pressure measuring apparatus may detect a pulse wave signal of an object in 1810, and may detect a contact pressure signal corresponding to a contact pressure between the object and the pulse wave sensor in operation 1820.

The blood pressure measuring apparatus may convert the pulse wave signal into an image by performing frequency domain conversion, to generate a pulse wave image in operation 1830, and may convert the contact pressure signal into an image to generate a contact pressure image in operation 1840.

The blood pressure measuring apparatus may obtain a plurality of contact images for a time frame in operation 1850.

The blood pressure measuring apparatus may convert a plurality of contact images for a time frame into a single contact image which represents a change in a contact image over the time frame in operation 1860. For example, the blood pressure measuring apparatus may convert a plurality of contact images for the time frame into a single contact image by: dividing a contact image of the time frame into a plurality of portions, arranging the portions in a predetermined arrangement order to generate arranged images of the contact image of the time frame, and arranging the generated arranged images in a time-sequential order.

The blood pressure measuring apparatus may estimate blood pressure of an object by using the pulse wave image, the contact pressure image, the single contact image, and the blood pressure estimation model in 1870. The blood pressure estimation model may be a model which is based on a convolutional neural network and is pre-generated by machine learning using pulse wave image data, contact pressure image data, single contact image data, and blood pressure data corresponding thereto as training data.

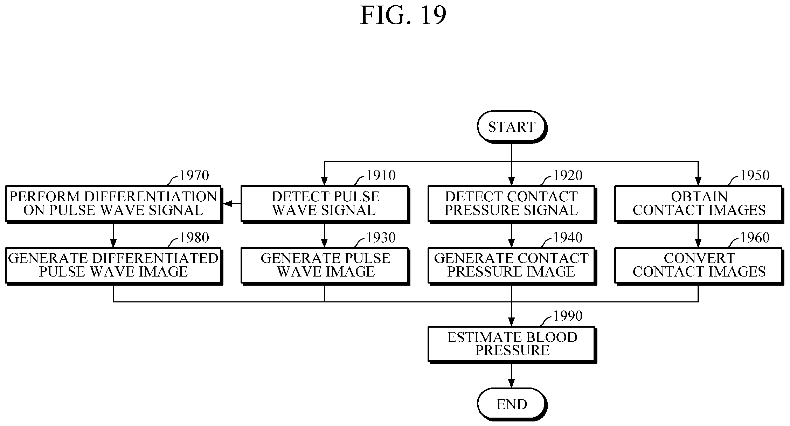

FIG. 19 is a flowchart illustrating still another example of a blood pressure measuring method according to an embodiment. The blood pressure measuring method of FIG. 19 may be performed by the blood pressure measuring apparatus 900 of FIG. 9. Operations 1910, 1920, 1930, 1940, 1950, and 1960 of FIG. 19 may be substantially similar as the operations 1810, 1820, 1830, 1840, 1850, and 1860, respectively of FIG. 18, and operations 1970 and 1980 of FIG. 19 may be substantially similar as the operations 1750 and 1760 respectively of FIG. 17, such that description thereof may be briefly made below.

Referring to FIG. 19, the blood pressure measuring apparatus may detect a pulse wave signal of an object in operation 1910, and may detect a contact pressure signal corresponding to a contact pressure between the object and the pulse wave sensor in operation 1920.

The blood pressure measuring apparatus may convert the pulse wave signal into an image by performing frequency domain conversion, to generate a pulse wave image in operation 1930, and may convert the contact pressure signal into an image to generate a contact pressure image in operation 1940.

The blood pressure measuring apparatus may obtain a plurality of contact images for a time frame in operation 1950, and may convert a plurality of contact images for the time frame into a single contact image which represents a change in a contact image over the time frame in operation 1960.

The blood pressure measuring apparatus may perform *n*th-order differentiation on the pulse wave signal to obtain the nth-order differentiated pulse wave signal in operation 1970, and may convert the obtained *n*th-order differentiated pulse wave signal into an image by performing frequency domain conversion, to generate an nth-order differentiated pulse wave image in operation 1980.

The blood pressure measuring apparatus may estimate blood pressure of an object by using the pulse wave image, the contact pressure image, the single contact image, the *n*th-order differentiated pulse wave image, and the blood pressure estimation model in operation 1990. The blood pressure estimation model may be a model which is based on a convolutional neural network and is pre-generated by machine learning using pulse wave image data, contact pressure image data, single contact image data, *n*th-order differentiated pulse wave image data, and blood pressure data corresponding thereto as training data.

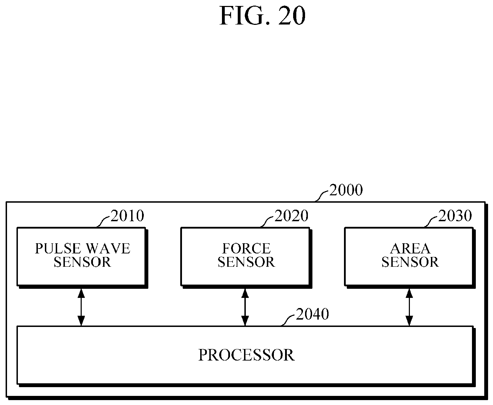

FIG. 20 is a block diagram illustrating still another example of a blood pressure measuring apparatus according to an embodiment. The blood pressure measuring apparatus 2000 of FIG. 20 is an apparatus for non-invasively measuring blood pressure of an object, and may be embedded in an electronic device or may be enclosed in a housing to be provided as a separate device.

Referring to FIG. 20, the blood pressure measuring apparatus 2000 includes a pulse wave sensor 2010, a force sensor 2020, an area sensor 2030, and a processor 2040. Here, the pulse wave sensor 2010 may be substantially similar as the pulse wave sensor 110 of FIG. 1, such that detailed description thereof may be omitted.

The force sensor 2020 may detect a contact force signal corresponding to a contact force between an object and the pulse wave sensor 2010. To this end, the force sensor 2020 may include a piezoelectric film, a load cell, radar, a strain gauge, and the like.

The area sensor 2030 may detect a contact area signal corresponding to a contact area between the object and the pulse wave sensor 2010. To this end, the area sensor 2030 may include a contact area sensor (e.g., touch sensor, etc.).

The processor 2040 may control the overall operation of the blood pressure measuring apparatus 2000.

Based on the object contacting the pulse wave sensor 2010, the processor 2040 may control the pulse wave sensor 2010 to detect a pulse wave signal of the object, may control the force sensor 2020 to detect a contact force signal corresponding to a contact force between the object and the pulse wave sensor 2010, and may control the area sensor 2030 to detect a contact area signal corresponding to a contact area between the object and the pulse wave sensor 2010.

Further, the processor 2040 may estimate blood pressure of the object based on the detected pulse wave signal, the detected contact force signal, and the detected contact area signal. For example, the processor 2040 may convert each of the pulse wave signal, the contact force signal, and the contact area signal into an image to generate a pulse wave image, a contact force image, and a contact area image, respectively, and may estimate blood pressure of the object by using the generated pulse wave image, contact force image, and contact area image.

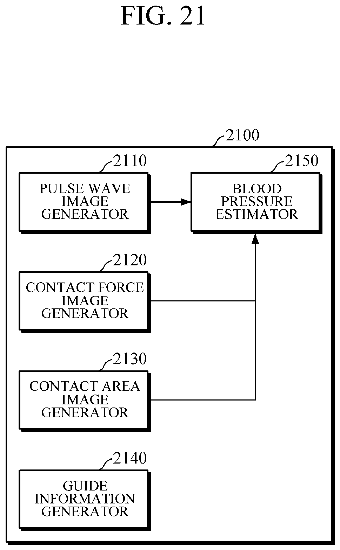

FIG. 21 is a block diagram illustrating an example of a processor according to an embodiment. The processor 2100 of FIG. 21 may be an example of the processor 2040 of FIG. 20.

Referring to FIG. 21, the processor 2100 includes a pulse wave image generator 2110, a contact force image generator 2120, a contact area image generator 2130, a guide information generator 2140, and a blood pressure estimator 2150. Here, the pulse wave image generator 2110 may be substantially similar as the pulse wave image generator 210 of FIG. 1, such that detailed description thereof may be omitted.

The contact force image generator 2120 may generate a contact force image by converting a contact force signal into an image. The contact force signal may be a non-periodic signal having an intensity which contains significant information. In an embodiment, the contact force image generator 2120 may generate a contact force image by converting a contact force signal into a gradient image. In this case, the generated contact force image may be represented in a time domain. That is, one axis (e.g., either a horizontal axis or a vertical axis) of the contact force image may represent a time domain reflecting physical properties with respect to time.

The contact area image generator 2130 may generate a contact area image by converting a contact area signal into an image. The contact area signal may be a non-periodic signal having an intensity which contains significant information. In an embodiment, the contact area image generator 2130 may generate a contact area image by converting a contact area signal into a gradient image. In this case, the generated contact area image may be represented in a time domain. That is, one axis (e.g., either a horizontal axis or a vertical axis) of the contact area image may represent a time domain reflecting physical properties with respect to time.

The guide information generator 2140 may obtain a contact pressure signal based on the detected contact force signal and the detected contact area signal; and based on the obtained contact pressure signal, the guide information generator 2140 may generate guide information that instructs a user to increase or decrease contact pressure between an object and the pulse wave sensor for measuring blood pressure, and may provide the generated guide information to a user via an output device. For example, the guide information generator 230 may obtain a contact pressure signal by dividing the contact force signal by the contact area signal. Further, the guide information generator 2140 may compare the obtained contact pressure signal with a target pressure signal which linearly increases or decreases; and in response to the obtained contact pressure signal being greater than the target pressure signal, the guide information generator 2140 may generate guide information that instructs a user to decrease the contact pressure, and in response to the measured contact pressure signal being less than the target pressure signal, the guide information generator 2140 may generate guide information that instructs a user to increase the contact pressure. Then, the guide information generator 2140 may provide the generated guide information to a user via an output device. In this case, examples of the output device may include various output devices such as a visual output device, an audio output device, a haptic output device, and the like.

The blood pressure estimator 2150 may estimate blood pressure of an object based on the pulse wave image, the contact force image, and the contact area image. In this case, the blood pressure estimator 2150 may use a pre-generated blood pressure estimation model. The blood pressure estimation model may be a model which is based on a convolutional neural network and is pre-generated by machine learning using pulse wave image data, contact force image data, contact area image data, and blood pressure data corresponding thereto as training data, and may be stored in an internal or external memory of the processor 2100.

In an embodiment, before estimating blood pressure, the blood pressure estimator 2150 may process the pulse wave image, the contact force image, and the contact area image so that the pulse wave image, the contact force image, and the contact area image may have the same size. For example, the blood pressure estimator 2150 may process the pulse wave image, the contact force image, and the contact area image so that the pulse wave image, the contact force image, and the contact area image may have the same time domain scale.

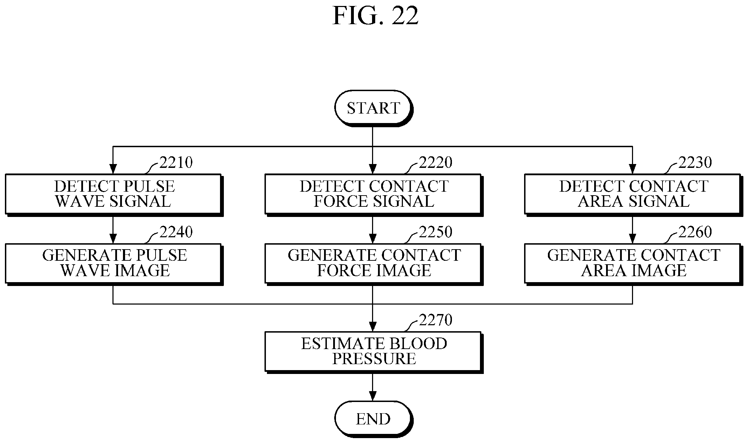

FIG. 22 is a flowchart illustrating still another example of a blood pressure measuring method according to an embodiment. The blood pressure measuring method of FIG. 22 may be performed by the blood pressure measuring apparatus 2000 of FIG. 20. Operations 2210 and 2240 of FIG. 22 may be substantially similar as the operations 1510 and 1530, respectively, of FIG. 15, such that description thereof may be briefly made below.

Referring to FIG. 22, the blood pressure measuring apparatus may detect a pulse wave signal of an object in operation 2210, may detect a contact force signal corresponding to a contact force between the object and the pulse wave sensor in operation 2220, and may detect a contact area signal corresponding to a contact area between the object and the pulse wave sensor in operation 2230.

The blood pressure measuring apparatus may convert the pulse wave signal into an image by performing frequency domain conversion, to generate a pulse wave image in operation 2240.

The blood pressure measuring apparatus may convert the contact force signal into an image to generate a contact force image in operation 2250. In an embodiment, the blood pressure measuring apparatus may generate a contact area image by converting the contact force signal into a gradient image.

The blood pressure measuring apparatus may convert the contact area signal into an image to generate a contact area image in operation 2260. In an embodiment, the blood pressure measuring apparatus may generate a contact area image by converting the contact area signal into a gradient image.

The blood pressure measuring apparatus may estimate blood pressure of an object based on the pulse wave image, the contact force image, the contact area image, and the blood pressure estimation model in operation 2270. The blood pressure estimation model may be a model which is based on a convolutional neural network and is pre-generated by machine learning using pulse wave image data, contact force image data, contact area image data, and blood pressure data corresponding thereto as training data.

While FIGS. 1 to 22 are examples of estimating blood pressure using the pulse wave signal, the contact pressure signal, the nth-order differentiated pulse wave signal, the contact images for the time frame, the contact force signal, and/or the contact area signal, the estimation of blood pressure is not limited thereto. That is, blood pressure may be estimated by further using a non-periodic signal and the like which reflect physical characteristics of a user such as temperature of an object, or measurement environments.

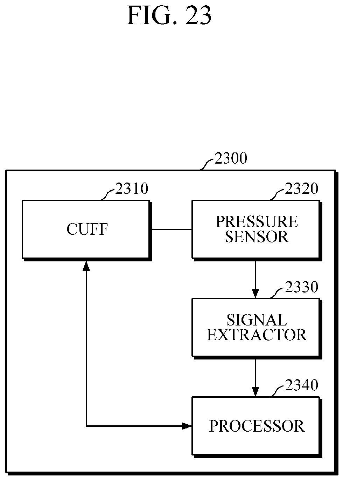

FIG. 23 is a block diagram illustrating a blood pressure measuring apparatus according to the present invention.

Referring to FIG. 23, the blood pressure measuring apparatus 2300 includes a cuff 2310, a pressure sensor 2320, a signal extractor 2330, and a processor 2340.

The cuff 2310 (e.g., a sphygmomanometer cuff) may be wrapped around an object to apply pressure to the object. Specifically, the cuff 2310 is placed around the upper arm of the object, and is inflated to a pressure higher than systolic blood pressure according to a predetermined control signal, so as to constrict the blood vessels near the upper arm, and then the pressure applied to the object is gradually decreased.

The pressure sensor 2320 detects a cuff pressure signal.

The signal extractor 2330 extracts an alternating current (AC) component signal and a direct current (DC) component signal from the measured cuff pressure signal. To this end, the signal extractor 2330 may include various filters such as a high-pass filter, a low-pass filter, and the like.

The processor 2340 may control the overall operation of the blood pressure measuring apparatus 2300.

Based on the cuff 2310 being placed around the object, the processor 2340 may control the cuff 2310 to apply pressure to the object, and may control the pressure sensor 2320 to detect a cuff pressure signal. Further, the processor 2340 may control the signal extractor 2330 to extract an AC component signal and a DC component signal from the detected cuff pressure signal.

The processor 2340 may estimate blood pressure of the object based on the extracted AC component signal and DC component signal. The processor 2340 converts each of the AC component signal and the DC component signal into an image, to generate an AC component image and a DC component image, and may estimate blood pressure of the object by using the generated AC component image and DC component image.

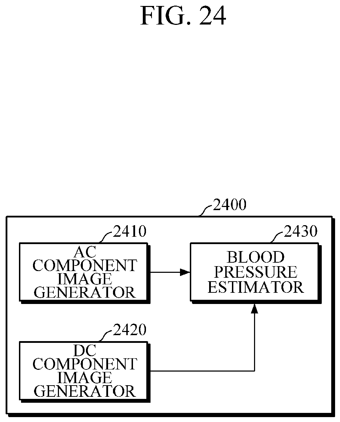

Hereinafter, the processor 2340 will be described in detail with reference to FIG. 24.

FIG. 24 is a block diagram illustrating an example of a processor according to an embodiment. The processor 2400 of FIG. 24 may be an example of the processor 2340 of FIG. 23.

Referring to FIG. 24, the processor 2400 includes an AC component image generator 2410, a DC component image generator 2420, and a blood pressure estimator 2430.

The AC component image generator 2410 may convert the AC component signal into an image by performing frequency domain conversion, to generate an AC component image. The AC component signal may be a periodic signal having a shape which contains significant information. In an embodiment, the AC component image generator 2410 may generate an AC component image by converting the AC component signal into an image using at least one of Wavelet Transform, Short Time Fourier transform, and Wigner-Ville distribution. In this case, the generated AC component image may be represented in a time domain and a frequency domain. That is, a horizontal axis of the AC component image represents a time domain which reflects physical properties with respect to time; and a vertical axis of the AC component image represents a frequency domain which reflects physical properties with respect to frequency. However, the AC component image is not limited thereto, and a horizontal axis may represent a frequency domain reflecting physical properties with respect to frequency, and a vertical axis may represent a time domain reflecting physical properties with respect to time.

The DC component image generator 2420 may generate a DC component image by converting the DC component signal into an image. The DC component signal may be a non-periodic signal having an intensity which contains significant information. In an embodiment, the DC component image generator 2420 may generate a DC component image by converting the DC component signal into a gradient image. In this case, the generated DC component image may be represented in a time domain. That is, one axis (e.g., either a horizontal axis or a vertical axis) of the DC component image may represent a time domain reflecting physical properties with respect to time.

The blood pressure estimator 2430 may estimate blood pressure of the object based on the AC component image and the DC component image. In this case, the blood pressure estimator 2430 may use a pre-generated blood pressure estimation model. The blood pressure estimation model may be a model which is based on a convolutional neural network and is pre-generated by machine learning using AC component image data, DC component image data, and blood pressure data corresponding thereto as training data, and may be stored in an internal or external memory of the processor 2400.

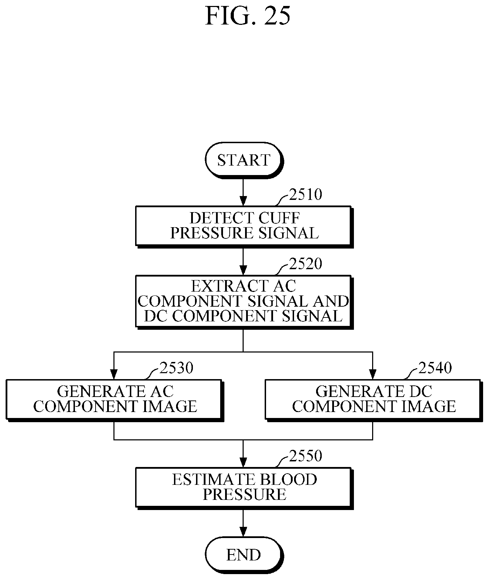

FIG. 25 is a flowchart illustrating a blood pressure measuring method according to the present invention. The blood pressure measuring method of FIG. 25 may be performed by the blood pressure measuring apparatus 2300 of FIG. 23.

Referring to FIG. 25, based on a cuff being placed around an object, the blood pressure measuring apparatus detects a cuff pressure signal in operation 2510.

The blood pressure measuring apparatus extracts an AC component signal and a DC component signal from the measured cuff pressure signal in operation 2520.

The blood pressure measuring apparatus converts the AC component signal into an image by performing frequency domain conversion, to generate an AC component image in 2530. The blood pressure measuring apparatus generates an AC component image by converting the AC component signal into an image using at least one of Wavelet Transform, Short Time Fourier transform, and Wigner-Ville distribution.

The blood pressure measuring apparatus generates a DC component image by converting the DC component signal into an image in operation 2540. In an embodiment, the blood pressure measuring apparatus generates a DC component image by converting the DC component signal into a gradient image.

The blood pressure measuring apparatus estimates blood pressure of the object by using the AC component image, the DC component image, and the blood pressure estimation model in operation 2550. The blood pressure estimation model may be a model which is based on a convolutional neural network and is pre-generated by machine learning using AC component image data, DC component image data, and blood pressure data corresponding thereto as training data.

The present disclosure is realized as computer-readable code stored on a non-transitory computer-readable medium. The computer-readable medium may be any type of recording device in which data is stored in a computer-readable manner. Examples of the computer-readable medium may include a ROM, a RAM, a CD-ROM, a magnetic tape, a floppy disc, an optical data storage, and a carrier wave (e.g., data transmission through the Internet). The computer-readable medium can be distributed via a plurality of computer systems connected to a network so that computer-readable code is written thereto and executed therefrom in a decentralized manner. Functional programs, codes, and code segments for implementing the present disclosure can be deduced by one of ordinary skill in the art.

The present disclosure has been described herein with regard to preferred embodiments. However, it will be obvious to those skilled in the art that various changes and modifications can be made without departing from the scope of the present disclosure. Thus, it is clear that the above-described embodiments are illustrative in all aspects and are not intended to limit the present disclosure.

## Claims

1. A blood pressure measuring method comprising:
based on a cuff (2310) being placed on an object, detecting (2510) a cuff pressure signal;
extracting (2520) an alternating current, AC, component signal and a direct current, DC, component signal based on the cuff pressure signal;
generating (2530) an AC component image based on the extracted AC component signal by converting the AC component signal into an image using at least one of Wavelet Transform, Short Time Fourier transform, and Wigner-Ville distribution;
generating (2540) a DC component image based on the extracted DC component signal by converting the DC component signal into a gradient image to generate the DC component image, representing the DC component signal over time; and
estimating (2550) a blood pressure of the object based on the generated AC component image, the generated DC component image, and a blood pressure estimation model;
said blood pressure estimation model being generated by machine learning based on training data, wherein the training data comprises AC component image data, DC component image data, and blood pressure data.

2. The blood pressure measuring method of claim 1 further comprising controlling the cuff (2310) to apply pressure to the object, and controlling the pressure sensor (2320) to detect the cuff pressure signal.

3. The blood pressure measuring method of claim 1, wherein the blood pressure estimation model is based on a convolutional neural network.

4. The blood pressure measuring method of claim 3, wherein the blood pressure estimation model is pre-generated by machine learning using the AC component image data, the DC component image data, and the blood pressure data.

5. The blood pressure measuring method of claim 1, wherein the generated AC component image is represented in a time domain and a frequency domain.

6. The blood pressure measuring method of claim 5, wherein a horizontal axis of the AC component image represents the time domain which reflects physical properties with respect to time, and a vertical axis of the AC component image represents the frequency domain which reflects physical properties with respect to frequency.

7. The blood pressure measuring method of claim 1, wherein the generated DC component image is represented in a time domain, wherein either a horizontal axis or a vertical axis of the DC component image is represented in the time domain reflecting physical properties with respect to time.

8. A blood pressure measuring apparatus comprising:
a cuff (2310) being placed on an object and configured to apply pressure to the object;
a pressure sensor (2320) configured to detect (2510) a cuff pressure signal;
a signal extractor (2330) configured to extract (2520) an alternating current, AC, component signal and a direct current, DC, component signal based on the cuff pressure signal; and
a processor (2340) configured to:
generate (2530) an AC component image based on the extracted AC component signal by converting the AC component signal into an image using at least one of Wavelet Transform, Short Time Fourier transform, and Wigner-Ville distribution;
generate (2540) a DC component image based on the extracted DC component signal by converting the DC component signal into a gradient image to generate the DC component image, representing the DC component signal over time; and
estimate (2550) a blood pressure of the object based on the generated AC component image, the generated DC component image, and a blood pressure estimation model,
wherein the blood pressure estimation model is generated by machine learning based on training data, wherein the training data comprises AC component image data, DC component image data, and blood pressure data.

9. The blood pressure measuring apparatus of claim 8, wherein the processor (3240) is further configured to control the cuff (2310) to apply pressure to the object, and to control the pressure sensor (2320) to detect the cuff pressure signal.

10. The blood pressure measuring apparatus of claim 8, wherein the blood pressure estimation model is based on a convolutional neural network.

11. The blood pressure measuring apparatus of claim 10, wherein the blood pressure estimation model is pre-generated by machine learning using the AC component image data, the DC component image data, and the blood pressure data.

12. The blood pressure measuring apparatus of claim 8, wherein the generated AC component image is represented in a time domain and a frequency domain.

13. The blood pressure measuring apparatus of claim 12, wherein a horizontal axis of the AC component image represents the time domain which reflects physical properties with respect to time, and a vertical axis of the AC component image represents the frequency domain which reflects physical properties with respect to frequency.

14. The blood pressure measuring apparatus of claim 8, wherein the generated DC component image is represented in a time domain, wherein either a horizontal axis or a vertical axis of the DC component image is represented in the time domain reflecting physical properties with respect to time.

15. A computer-readable medium having instructions that, when performed by a processor (2340) of a blood pressure measuring apparatus having a cuff (2310) being placed on an object and configured to apply pressure to the object, a pressure sensor (2320) configured to detect (2510) a cuff pressure signal, a signal extractor (2330) configured to extract (2520) an alternating current, AC, component signal and a direct current, DC, component signal based on the cuff pressure signal, cause the processor (2340) to:
generate (2530) an AC component image based on the extracted AC component signal by converting the AC component signal into an image using at least one of Wavelet Transform, Short Time Fourier transform, and Wigner-Ville distribution;
generate (2540) a DC component image based on the extracted DC component signal by converting the DC component signal into a gradient image to generate the DC component image, representing the DC component signal over time; and
estimate (2550) a blood pressure of the object based on the generated AC component image, the generated DC component image, and a blood pressure estimation model,
wherein the blood pressure estimation model is generated by machine learning based on training data, wherein the training data comprises AC component image data, DC component image data, and blood pressure data.

## Patentansprüche

1. Blutdruckmessverfahren, umfassend:
basierend auf dem Anlegen einer Manschette (2310) an ein Objekt, Detektieren (2510) eines Manschettendrucksignals;
Extrahieren (2520) eines Wechselstrom, AC, -Komponentensignals und eines Gleichstrom, DC, -Komponentensignals basierend auf dem Manschettendrucksignal;
Erzeugen (2530) eines AC-Komponentenbildes basierend auf dem extrahierten AC-Komponentensignal durch Umwandeln des AC-Komponentensignals in ein Bild unter Verwendung von mindestens einer Wavelet-Transformation, Kurzzeit-Fouriertransformation und Wigner-Ville-Verteilung;
Erzeugen (2540) eines DC-Komponentenbildes basierend auf dem extrahierten DC-Komponentensignal durch Umwandeln des DC-Komponentensignals in ein Gradientenbild, um das DC-Komponentenbild zu erzeugen, das das DC-Komponentensignal über die Zeit repräsentiert; und
Schätzen (2550) eines Blutdrucks des Objekts basierend auf dem erzeugten AC-Komponentenbild, dem erzeugten DC-Komponentenbild und einem Blutdruckschätzmodell,
wobei das Blutdruckschätzmodell durch maschinelles Lernen basierend auf Trainingsdaten erzeugt wird, wobei die Trainingsdaten AC-Komponentenbilddaten, DC-Komponentenbilddaten und Blutdruckdaten umfassen.

2. Blutdruckmessverfahren nach Anspruch 1 ferner umfassend das Steuern der Manschette (2310), um Druck auf das Objekt auszuüben, und das Steuern des Drucksensors (2320), um das Manschettendrucksignal zu detektieren.

3. Blutdruckmessverfahren nach Anspruch 1, wobei das Blutdruckschätzmodell auf einem neuronalen Faltungsnetzwerk basiert.

4. Blutdruckmessverfahren nach Anspruch 3, wobei das Blutdruckschätzmodell durch maschinelles Lernen unter Verwendung der AC-Komponentenbilddaten, der DC-Komponentenbilddaten und der Blutdruckdaten vorerzeugt wird.

5. Blutdruckmessverfahren nach Anspruch 1, wobei das erzeugte AC-Komponentenbild in einem Zeitbereich und einem Frequenzbereich repräsentiert wird.

6. Blutdruckmessverfahren nach Anspruch 5, wobei eine horizontale Achse des AC-Komponentenbildes den Zeitbereich repräsentiert, der physikalische Eigenschaften in Bezug auf die Zeit wiedergibt, und eine vertikale Achse des AC-Komponentenbildes den Frequenzbereich repräsentiert, der physikalische Eigenschaften in Bezug auf die Frequenz wiedergibt.

7. Blutdruckmessverfahren nach Anspruch 1, wobei das erzeugte DC-Komponentenbild in einem Zeitbereich dargestellt wird, wobei entweder eine horizontale Achse oder eine vertikale Achse des DC-Komponentenbildes in dem Zeitbereich dargestellt wird, der physikalische Eigenschaften in Bezug auf die Zeit wiedergibt.

8. Blutdruckmessvorrichtung, umfassend:
eine Manschette (2310), die an einem Objekt angelegt wird und konfiguriert ist, zum Ausüben von Druck auf das Objekt;
einen Drucksensor (2320) konfiguriert zum Detektieren (2510) eines Manschettendrucksignals;
einen Signalextraktor (2330) konfiguriert zum Extrahieren (2520) eines Wechselstrom, AC, -Komponentensignals und eines Gleichstrom, DC, -Komponentensignals, basierend auf dem Manschettendrucksignal; und
einen Prozessor (2340), konfiguriert zum:
Erzeugen (2530) eines AC-Komponentenbildes basierend auf dem extrahierten AC-Komponentensignal durch Umwandeln des AC-Komponentensignals in ein Bild unter Verwendung von mindestens einer Wavelet-Transformation, Kurzzeit-Fouriertransformation und Wigner-Ville-Verteilung;
Erzeugen (2540) eines DC-Komponentenbildes basierend auf dem extrahierten DC-Komponentensignal durch Umwandeln des DC-Komponentensignals in ein Gradientenbild, um das DC-Komponentenbild zu erzeugen, das das DC-Komponentensignal über die Zeit repräsentiert; und
Schätzen (2550) eines Blutdrucks des Objekts basierend auf dem erzeugten AC-Komponentenbild, dem erzeugten DC-Komponentenbild und einem Blutdruckschätzmodell,
wobei das Blutdruckschätzmodell durch maschinelles Lernen basierend auf Trainingsdaten erzeugt wird, wobei die Trainingsdaten AC-Komponentenbilddaten, DC-Komponentenbilddaten und Blutdruckdaten umfassen.

9. Blutdruckmessvorrichtung nach Anspruch 8, wobei der Prozessor (3240) ferner konfiguriert ist, zum Steuern der Manschette (2310), um Druck auf das Objekt auszuüben, und zum Steuern des Drucksensors (2320), um das Manschettendrucksignal zu detektieren.

10. Blutdruckmessvorrichtung nach Anspruch 8, wobei das Blutdruckschätzmodell auf einem neuronalen Faltungsnetzwerk basiert.

11. Blutdruckmessvorrichtung nach Anspruch 10, wobei das Blutdruckschätzmodell durch maschinelles Lernen unter Verwendung der AC-Komponentenbilddaten, der DC-Komponentenbilddaten und der Blutdruckdaten vorerzeugt wird.

12. Blutdruckmessvorrichtung nach Anspruch 8, wobei das erzeugte AC-Komponentenbild in einem Zeitbereich und einem Frequenzbereich repräsentiert wird.

13. Blutdruckmessvorrichtung nach Anspruch 12, wobei eine horizontale Achse des AC-Komponentenbildes den Zeitbereich repräsentiert, der physikalische Eigenschaften in Bezug auf die Zeit wiedergibt, und eine vertikale Achse des AC-Komponentenbildes den Frequenzbereich repräsentiert, der physikalische Eigenschaften in Bezug auf die Frequenz wiedergibt.

14. Blutdruckmessvorrichtung nach Anspruch 8, wobei das erzeugte DC-Komponentenbild in einem Zeitbereich dargestellt wird, wobei entweder eine horizontale Achse oder eine vertikale Achse des DC-Komponentenbildes in dem Zeitbereich dargestellt wird, der physikalische Eigenschaften in Bezug auf die Zeit wiedergibt.

15. Computerlesbares Medium mit Instruktionen, die, wenn sie von einem Prozessor (2340) einer Blutdruckmessvorrichtung ausgeführt werden, mit einer Manschette (2310), die auf einem Objekt platziert wird und konfiguriert ist, zum Ausüben von Druck auf das Objekt, einen Drucksensor (2320), der konfiguriert ist, zum Detektieren (2510) eines Manschettendrucksignals, einen Signalextraktor (2330), der konfiguriert ist, zum Extrahieren (2520) eines Wechselstrom, AC-, Komponentensignals und eines Gleichstrom, DC-, Komponentensignals basierend auf dem Manschettendrucksignal, den Prozessor (2340) veranlassen, zum:
Erzeugen (2530) eines AC-Komponentenbildes basierend auf dem extrahierten AC-Komponentensignal durch Umwandeln des AC-Komponentensignals in ein Bild unter Verwendung von mindestens einer Wavelet-Transformation, Kurzzeit-Fouriertransformation und Wigner-Ville-Verteilung;
Erzeugen (2540) eines DC-Komponentenbildes basierend auf dem extrahierten DC-Komponentensignal durch Umwandeln des DC-Komponentensignals in ein Gradientenbild, um das DC-Komponentenbild zu erzeugen, das das DC-Komponentensignal über die Zeit repräsentiert; und
Schätzen (2550) eines Blutdrucks des Objekts basierend auf dem erzeugten AC-Komponentenbild, dem erzeugten DC-Komponentenbild und einem Blutdruckschätzmodell,
wobei das Blutdruckschätzmodell durch maschinelles Lernen basierend auf Trainingsdaten erzeugt wird, wobei die Trainingsdaten AC-Komponentenbilddaten, DC-Komponentenbilddaten und Blutdruckdaten umfassen.

## Revendications

1. Procédé de mesure de pression artérielle comprenant :
selon un brassard (2310) placé sur un objet, une détection (2510) d'un signal de pression de brassard ;
une extraction (2520) d'un signal de composante de courant alternatif, CA, et d'un signal de composante de courant continu, CC, selon le signal de pression de brassard ;
une génération (2530) d'une image de composante CA basée sur le signal de composante CA extrait en convertissant le signal de composante CA en une image au moyen d'au moins une d'une transformée en ondelettes, d'une transformée de Fourier à court terme et d'une distribution Wigner-Ville ;
une génération (2540) d'une image de composante CC basée sur le signal de composante CC extrait en convertissant le signal de composante CC en une image de gradient pour générer l'image de composante CC, représentant le signal de composante CC dans le temps ; et
une estimation (2550) d'une pression artérielle de l'objet basée sur l'image de composante CA générée, l'image de composante CC générée et un modèle d'estimation de pression artérielle ;
ledit modèle d'estimation de pression artérielle étant généré par un apprentissage machine basé sur des données d'entraînement, dans lequel les données d'entraînement comprennent des données d'image de composante CA, des données d'image de composante CC et des données de pression artérielle.

2. Le procédé de mesure de pression artérielle de la revendication 1 comprenant en outre une commande du brassard (2310) pour appliquer une pression à l'objet, et une commande du capteur de pression (2320) pour détecter le signal de pression de brassard.

3. Le procédé de mesure de pression artérielle de la revendication 1, dans lequel le modèle d'estimation de pression artérielle repose sur un réseau neuronal à convolution.

4. Le procédé de mesure de pression artérielle de la revendication 3, dans lequel le modèle d'estimation de pression artérielle est pré-généré par apprentissage machine en utilisant les données d'image de composante CA, les données d'image de composante CC et les données de pression artérielle.

5. Le procédé de mesure de pression artérielle de la revendication 1, dans lequel l'image de composante CA générée est représentée dans un domaine temporel et un domaine fréquentiel.

6. Le procédé de mesure de pression artérielle de la revendication 5, dans lequel un axe horizontal de l'image de composante CA représente le domaine temporel qui reflète des propriétés physiques par rapport au temps, et un axe vertical de l'image de composante CA représente le domaine fréquentiel qui reflète des propriétés physiques par rapport à une fréquence.

7. Le procédé de mesure de pression artérielle de la revendication 1, dans lequel l'image de composante CC générée est représentée dans un domaine temporel, dans lequel soit un axe horizontal, soit un axe vertical de l'image de composante CC est représenté dans le domaine temporel reflétant des propriétés physiques par rapport au temps.

8. Appareil de mesure de pression artérielle comprenant :
un brassard (2310) placé sur un objet et configuré pour appliquer une pression à l'objet ;
un capteur de pression (2320) configuré pour détecter (2510) un signal de pression de brassard ;
un extracteur de signal (2330) configuré pour extraire (2520) un signal de composante de courant alternatif, CA, et d'un signal de composante de courant continu, CC, selon le signal de pression de brassard ; et
un processeur (2340) configuré pour :
générer (2530) une image de composante CA basée sur le signal de composante CA extrait en convertissant le signal de composante CA en une image au moyen d'au moins une d'une transformée en ondelettes, d'une transformée de Fourier à court terme et d'une distribution Wigner-Ville ;
générer (2540) une image de composante CC basée sur le signal de composante CC extrait en convertissant le signal de composante CC en une image de gradient pour générer l'image de composante CC, représentant le signal de composante CC dans le temps ; et
estimer (2550) une pression artérielle de l'objet basée sur l'image de composante CA générée, l'image de composante CC générée et un modèle d'estimation de pression artérielle,
dans lequel le modèle d'estimation de pression artérielle est généré par un apprentissage machine basé sur des données d'entraînement, dans lequel les données d'entraînement comprennent des données d'image de composante CA, des données d'image de composante CC et des données de pression artérielle.

9. L'appareil de mesure de pression artérielle de la revendication 8, dans lequel le processeur (3240) est configuré en outre pour commander le brassard (2310) pour appliquer une pression à l'objet, et pour commander le capteur de pression (2320) pour détecter le signal de pression de brassard.

10. Le procédé de mesure de pression artérielle de la revendication 8, dans lequel le modèle d'estimation de pression artérielle repose sur un réseau neuronal à convolution.

11. L'appareil de mesure de pression artérielle de la revendication 10, dans lequel le modèle d'estimation de pression artérielle est pré-généré par apprentissage machine en utilisant les données d'image de composante CA, les données d'image de composante CC et les données de pression artérielle.

12. L'appareil de mesure de pression artérielle de la revendication 8, dans lequel l'image de composante CA générée est représentée dans un domaine temporel et un domaine fréquentiel.

13. L'appareil de mesure de pression artérielle de la revendication 12, dans lequel un axe horizontal de l'image de composante CA représente le domaine temporel qui reflète des propriétés physiques par rapport au temps, et un axe vertical de l'image de composante CA représente le domaine fréquentiel qui reflète des propriétés physiques par rapport à une fréquence.

14. L'appareil de mesure de pression artérielle de la revendication 8, dans lequel l'image de composante CC générée est représentée dans un domaine temporel, dans lequel soit un axe horizontal, soit un axe vertical de l'image de composante CC est représenté dans le domaine temporel reflétant des propriétés physiques par rapport au temps.

15. Support lisible par un ordinateur renfermant des instructions qui, lorsqu'exécutées par un processeur (2340) d'un appareil de mesure de pression artérielle présentant un brassard (2310) placé sur un objet et configuré pour appliquer une pression à l'objet, un capteur de pression (2320) configuré pour détecter (2510) un signal de pression de brassard, un extracteur de signal (2330) configuré pour extraire (2520) un signal de composante de courant alternatif, CA, et un signal de composante de courant continu, CC, selon le signal de pression de brassard, font exécuter par le processeur (2340) :
une génération (2530) d'une image de composante CA basée sur le signal de composante CA extrait en convertissant le signal de composante CA en une image au moyen d'au moins une d'une transformée en ondelettes, d'une transformée de Fourier à court terme et d'une distribution Wigner-Ville ;
une génération (2540) d'une image de composante CC basée sur le signal de composante CC extrait en convertissant le signal de composante CC en une image de gradient pour générer l'image de composante CC, représentant le signal de composante CC dans le temps ; et
une estimation (2550) d'une pression artérielle de l'objet basée sur l'image de composante CA générée, l'image de composante CC générée et un modèle d'estimation de pression artérielle,
dans lequel le modèle d'estimation de pression artérielle est généré par un apprentissage machine basé sur des données d'entraînement, dans lequel les données d'entraînement comprennent des données d'image de composante CA, des données d'image de composante CC et des données de pression artérielle.
